# EUROPÄISCHE PATENTSCHRIFT

(11) **EP 3 448 153 B1**
(45) Veröffentlichungstag und Bekanntmachung des Hinweises auf die Patenterteilung: **28.04.2021**
(21) Anmeldenummer: 17719154.1
(22) Anmeldetag: 31.03.2017
(51) Int. Cl.: A01N 1/02, G01K 3/04, G01K 11/06

(54) **VORRICHTUNG UND VERFAHREN ZUR TEMPERATURÜBERWACHUNG EINER KRYOKONSERVIERTEN BIOLOGISCHEN PROBE**
DEVICE AND METHOD FOR MONITORING THE TEMPERATURE OF A CRYOPRESERVED BIOLOGICAL SAMPLE
DISPOSITIF ET PROCÉDÉ DISPOSITIF DE SURVEILLANCE DE LA TEMPÉRATURE D'UN ÉCHANTILLON BIOLOGIQUE CRYOCONSERVÉ

(30) Priorität: 27.04.2016 DE 102016005070
(43) Veröffentlichungstag der Anmeldung: 06.03.2019
(73) Patentinhaber: Fraunhofer-Gesellschaft zur Förderung der angewandten Forschung e.V., 80686 München (DE)
(72) Erfinder: FUHR, Günther R., 13187 Berlin (DE); ZIMMERMANN, Heiko, 97295 Waldbrunn (DE)
(74) Vertreter: v. Bezold & Partner Patentanwälte - PartG mbB
(86) Internationale Anmeldenummer: PCT/EP2017/000401
(87) Internationale Veröffentlichungsnummer: WO 2017/186328

(56) Entgegenhaltungen:
- WO-A2-2008/027814
- CN-B- 103 630 503
- US-A- 4 134 359
- Anonymous: "CryoguardTM - Key Information", , 18. April 2016 (2016-04-18), XP055383714, Gefunden im Internet: URL:https://web.archive.org/web/2016041817 1529/http://www.cryoguard.com/key-informat ion/ [gefunden am 2017-06-21]

## Beschreibung

Die Erfindung betrifft eine Vorrichtung zur Temperaturüberwachung einer kryokonservierten biologischen Probe. Die Erfindung betrifft ferner ein Verfahren zur Temperaturüberwachung einer kryokonservierten biologischen Probe.

Die Tieftemperaturkonservierung (Kryokonservierung) von Zellen ist bisher die einzige Möglichkeit, Lebensprozesse auf zellulärer Ebene reversibel (vitalitätserhaltend) so anzuhalten, dass sie nach einer Erwärmung auf physiologische Temperaturen wieder anlaufen können. Die Kryokonservierung hat sich über große Biobanken in den letzten Jahrzehnten zu einem unverzichtbaren Element für Kliniken, Pharmaunternehmen, die Arterhaltung, den Umweltschutz und die Gesundheitsvorsorge entwickelt. Gelagert wird biologisches Material in tieftemperaturverträglichen Probenbehältern (Kryobehältern), z. B. Röhrchen, Straws und Beuteln, unterschiedlicher Größe. Bei der Kryokonservierung sind die gelagerten Biomaterialien unter Aufrechterhaltung der Vitalität des Probenmaterials gefroren, zumeist bei Temperaturen unterhalb -80 °C, für Lebendsammlungen unter-140 °C bis zur Temperatur des flüssigen Stickstoffs. Für eine kryokonservierte Probe oder eine für die Kryokonservierung vorgesehene Probe wird nachfolgend auch der Begriff "Kryoprobe" verwendet.

Für makroskopische Proben, wie z. B. Blut oder Gewebe, sind zahlreiche Techniken zur Probenlagerung bei tiefen Temperaturen entwickelt worden. In der modernen Medizin, Gentechnik und Biologie besteht die Tendenz, zunehmend kleine Proben einer Kryokonservierung zu unterziehen. Es werden beispielsweise kleine Suspensionsvolumina (Milliliter oder darunter) mit suspendierten Zellen oder Zellgruppen eingefroren. Die Kryokonservierung von Zellen aus In-vitro-Kulturen erfolgt in überwiegendem Maße in einer Suspension. Die meisten der biomedizinisch relevanten Zellen benötigen jedoch zu ihrer Vermehrung und geordneten Entwicklung einen Substratkontakt. Daher werden Proben ggf. nach einer Kultivierung im substratgebundenen Zustand eingefroren.

Die Qualität der Proben ist von ausschlaggebender Bedeutung, da sie für Zelltherapien in Kliniken, die Entwicklung von Pharmaka und biotechnologischen Produkten, als nationale Ressourcen und vieles mehr Anwendung finden. Die Lagerzeit liegt bei einigen Tagen bis zu Jahrzehnten, mit einer Tendenz zur Langzeitlagerung. Die Proben werden in gekühlten Behältern gelagert, befinden sich zumeist in Metalleinschüben und Racks, mit denen sie bei neuen Einlagerungen oder Entnahmen Temperaturschwankungen unterliegen. Bei Lebendablagen (Zellen, Zellsuspensionen und Gewebeteilen) spielt nicht nur die ununterbrochene Kühlkette eine entscheidende Rolle, sondern auch die Vermeidung großer Temperatursprünge in der Tiefkühlphase. Da es bei der Entnahme gar nicht so selten vorkommt, dass Kryobehälter sich auf Temperaturen von -80 °C bis -20 °C erwärmen, treten, obwohl sie noch gefroren sind, Qualitätsminderungen unerkannt auf, die nicht nur den Wert der Probe mindern, sondern auch bei ihrer Verwendung im klinischen Bereich zu lebensgefährlichen Situationen führen können. Selbst kurzzeitig aufgetauten Proben sieht man im wiedergefrorenen Zustand nicht an, dass sie dem Originalzustand nicht mehr entsprechen. Es geht aber vornehmlich nicht nur darum, ein Auftauen der Biomaterialien zu erkennen, sondern das Überschreiten einer Grenztemperatur im Bereich zwischen -140 °C und -20 °C zu dokumentieren. Eine Temperaturkontrolle und -dokumentation für jede Probe ist die Forderung und bislang nur selten - und wenn, dann mit hohem technischen Aufwand - zu erfüllen. Hinzu kommen umfangreiche Laboruntersuchungen nach dem Auftauen, die ebenfalls wertvolles Probenmaterial verbrauchen und selbst im Falle inzwischen wertlos gewordener Kryoproben Kosten erzeugen.

US4134359 offenbart einen Kryostraw, dessen Innenraum zusätzlich zur biologischen Probe eine gefärbte Indikatorsubstanz enthält, deren Schmelztemperatur oberhalb der gewählten kryogenen Lagerungstemperatur liegt, z.B.bei -99 °C. Die Indikatorsubstanz ist von der Probe durch eine Barriere getrennt, z.B. Baumwolle, welche für die Indikatorsubstanz im geschmolzenen Zustand permeabel ist.

J Es ist somit eine Aufgabe der Erfindung, ein verbessertes Verfahren zur Temperaturüber-wachung einer kryokonservierten biologischen Probe bereitzustellen, mit dem Nachteile herkömmlicher Techniken vermieden werden können und das sich durch eine vereinfachte Verfahrensführung auszeichnet. Eine weitere Aufgabe ist es, eine Vorrichtung zur Temperaturüberwachung einer kryokonservierten biologischen Probe bereitzustellen, mit der Nachteile herkömmlicher Techniken vermieden werden können.

Eine weitere Aufgabe ist es, eine Möglichkeit bereitzustellen, um an einem möglichst einfachen Marker oder Kennzeichen erkennen zu können, ob eine Kryoprobe sich über eine definierbare Grenztemperatur, und wenn auch nur kurzzeitig, erwärmt hat. Die Grenztemperatur muss im Bereich zwischen -20 °C und -140 °C vor dem Einfrieren festlegbar sein. Dies sollte an jeder einzelnen Kryoprobe und an damit Millionen von Proben rasch und leicht erkennbar möglich sein, darf die Biomaterialien nicht verändern und sollte bereits im tiefgefrorenen Zustand erfolgen. Wenn möglich, sollte der Zustand der Probe auch im Lagerbehälter erfassbar sein, da jede Aus- und Einlagerung die Gefahr der Probenveränderung einer Vielzahl von Proben im Lagergut mit sich bringt, da in der Regel ganze Racks aufgezogen werden. Die Vorrichtung bzw. das Verfahren sollte leicht handhabbar, tieftemperaturtolerant und einstellbar sein. Es darf nur wenig oder keine Energie verbrauchen und möglichst nur geringste Kosten verursachen, da die Lagerung einer Bioprobe im gekühlten Zustand in ihren Gesamtaufwendungen nur wenige Euros kosten sollte. Diesem Anspruch müssen auch die einsetzbaren Materialien gerecht werden. Es wäre weiterhin wünschenswert, wenn nicht nur die Überschreitung einer zu überwachenden Grenztemperatur, sondern auch ein Maß für die Zeitdauer dieser Überschreitung erfasst werden könnte.

Diese Aufgaben werden durch Vorrichtungen und Verfahren mit den Merkmalen der unabhängigen Ansprüche gelöst. Vorteilhafte Ausführungsformen und Anwendungen der Erfindung ergeben sich aus den abhängigen Ansprüchen und werden in der folgenden Beschreibung unter teilweiser Bezugnahme auf die Figuren näher erläutert.

Gemäß einem ersten Gesichtspunkt der Erfindung werden die genannten Aufgaben durch eine Vorrichtung zur Temperaturüberwachung einer kryokonservierten biologischen Probe gelöst. Die Vorrichtung umfasst einen Probenbehälter mit einem Aufnahmeraum (Probenreservoir) zur Aufnahme einer biologischen Probe. Der Probenbehälter ist insbesondere ein Kryoprobenbehälter. Bei Verwendung der Vorrichtung kann der Aufnahmehohlraum eine kryokonservierte Probe enthalten.

Die Vorrichtung umfasst ferner mindestens eine Kammer, deren Innenraum mit dem Aufnahmeraum nicht fluidisch verbunden ist und lediglich zum Teil mit einer Indikatorsubstanz gefüllt ist, deren Schmelztemperatur bei Normaldruck, also bei 1013,25 hPa, in einem Bereich von -20 °C bis -140 °C liegt. Die Schmelztemperatur kann vorzugsweise auch in einem Bereich von -20 °C bis -100 °C liegen. Hierbei weist die Kammer eine Barriere auf, die bewirkt, dass, wenn sich Indikatorsubstanz in einem ersten Teilbereich der Kammer im flüssigen Aggregatszustand befindet, diese Indikatorsubstanz verzögert, d. h. zeitlich retardiert, in einen zweiten Teilbereich der Kammer gelangt. Die Barriere ist für die Indikatorsubstanz, wenn diese gefroren ist, nicht durchlässig. Die Barriere dient somit als ein Verzögerungsmittel, das ausgelegt ist, einen Flüssigkeitsstrom verzögert aus dem ersten Teilbereich der Kammer in den zweiten Teilbereich zu lassen.

Die erfindungsgemäße Vorrichtung weist außerdem die Merkmale der Variante i) oder der Variante ii) des Anspruchs 1 auf.

Durch die Kammer der erfindungsgemäßen Vorrichtung wird ein Zusatzkompartiment bereitgestellt, das durch die partielle Füllung mit der Indikatorsubstanz als Indikatorelement bzw. Indikatoreinrichtung verwendet werden kann, um eine unerwünschte Überschreitung der Grenztemperatur anzuzeigen. Wird eine zu überwachende Grenztemperatur, die der Schmelztemperatur der Indikatorsubstanz oder einer oberhalb der Schmelztemperatur liegenden Schwellentemperatur, bei der eine Viskosität der flüssig geworden Indikatorsubstanz genügend stark abgenommen hat, entspricht, überschritten, wird die Indikatorsubstanz flüssig und gelangt von dem ersten Teilbereich in den zweiten Teilbereich der Kammer. Befindet sich somit zumindest ein Teil der Indikatorsubstanz zu einem späteren Kontrollzeitpunkt in dem zweiten Teilbereich, kann auf eine zwischenzeitlich erfolgte, zumindest kurzzeitige Überschreitung der Grenztemperatur geschlossen werden.

Ein besonderer Vorzug der erfindungsgemäßen Vorrichtung ist ferner, dass aufgrund der Barriere die Indikatorsubstanz beim Flüssigwerden nicht sofort vollständig in den zweiten Teilbereich der Kammer gelangt, sondern dies verzögert erfolgt und somit hierfür eine vorbestimmte Zeit in Anspruch nimmt. Daher kann aus der Menge an Indikatorsubstanz, die sich zu einem bestimmten Zeitpunkt in dem zweiten Teilbereich befindet, ein Maß für die Zeitdauer einer zurückliegenden Überschreitung der Schmelztemperatur abgeleitet werden. Hierbei kann beispielsweise vorab empirisch durch Versuche der Zusammenhang zwischen Menge an Indikatorsubstanz und Zeitdauer der Überschreitung der Schmelztemperatur ermittelt werden und beispielsweise in Form einer Skala an der Kammerwandung bereitgestellt werden. Die Skala kann die entsprechende Zeitdauer der Überschreitung der Schmelztemperatur anzeigen, z. B. in Abhängigkeit von einem Füllstande der Indikatorsubstanz in einem der Teilbereiche oder in Abhängigkeit von der Länge der Diffusionsstrecke im zweiten Teilbereich.

Gemäß einer bevorzugten Ausführungsform kann somit eine Wandung des zweiten Teilbereichs der Kammer und/oder eine Wandung des ersten Teilbereichs der Kammer eine Skala zur Anzeige einer Zeitdauer der Überschreitung der Schmelztemperatur aufweisen.

Gemäß einem weiteren Aspekt können eine Wandung des zweiten Teilbereichs der Kammer und/oder eine Wandung des ersten Teilbereichs der Kammer an mindestens einer Stelle transparent oder semi-transparent sein. Dadurch kann einfacher bestimmt werden, ob sich Indikatorsubstanz im zweiten Teilbereich befindet, und/oder der Füllstand in zumindest einem der beiden Teilbereiche bestimmt werden. Ferner kann die gesamte Kammer auch aus einem transparenten oder semi-transparenten Material gefertigt sein, d. h., die Kammer kann von außen einsehbar sein.

Zur besseren Erkennbarkeit von Konfigurationsänderungen kann die Indikatorsubstanz einen Indikatorzusatz enthalten, der eine Detektierbarkeit einer physikalischen Eigenschaft der Indikatorsubstanz erhöht. Der Indikatorzusatz kann beispielsweise ein Farbstoff sein, so dass die Indikatorsubstanz farbig oder gefärbt, d. h. nicht transparent, ist und so deren Vorhandensein im zweiten Teilbereich besser optisch erkennbar ist.

Als Farbstoff kommt grundsätzlich jeder Farbstoff in Frage, welcher mindestens die folgenden Bedingungen erfüllt:
- intensives Färbevermögen auch in kleinen Mengen und Konzentrationen (z. B. ausgehend von einer gesättigten Farblösung Zugabe im Bereich < 1 Volumen-%, in der Regel im Promille- oder Subpromille-Bereich).
- frosttolerant
- lichtecht bei den Versand- als auch den relevanten tiefen Temperaturen
- löslich in allen Bestandteilen der Indikatorsubstanz
- kein Entmischen beim Einfrieren
- keine Reaktion mit Kunststoffmaterialien, welche in Kontakt mit der Indikatorsubstanz kommen.

Vorzugsweise ist der Farbstoff aus der Gruppe ausgewählt, welche Triphenylmethanfarbstoffe, Rhodaminfarbstoffe, insbesondere Xanthene, Azofarbstoffe sowie Phenazin- und Phenothiazinfarbstoffe umfasst.

In spezielleren Ausführungsformen ist der Farbstoff aus der Gruppe ausgewählt, welche Oil Red, Methylrot, Brillantgrün, Rhodamin B, Neutralrot, Methylenblau oder andere Farbstoffe, die zur Anfärbung von Zellen in der Zytologie verwendet werden, umfasst.

Der Indikatorzusatz können Partikel, insbesondere Nanopartikel sein, die eine Streuwirkung und/oder Polarisationswirkung der Indikatorsubstanz für auf die Indikatorsubstanz auftreffende elektromagnetische Strahlung erhöhen. Dadurch kann z. B. ein Füllstand der Indikatorsubstanz im zweiten Teilbereich mittels einer optischen Transmissionsmessung, Streumessung und/oder Polarisationsmessung zuverlässiger detektiert werden. Der Indikatorzusatz können leitfähige Partikel sein. Durch Beimischen von leitfähigen Partikel kann die Leitfähigkeit oder Impedanz der Indikatorsubstanz beeinflusst werden. Auf diese Weise kann das Vorhandensein der Indikatorsubstanz mittels einer Leitfähigkeitsmessung oder Impendanzmessung detektiert werden. Aufgrund des beigemischten Indikatorzusatzes kann die entsprechende Eigenschaft der Indikatorsubstanz mit einer entsprechend zweckmäßig ausgebildeten Messeinrichtung erfasst werden, um das Vorhandensein und/oder einen Füllstand der Indikatorsubstanz in dem ersten und/oder zweiten Teilbereich zuverlässiger bestimmen zu können.

Der Probenbehälter ist ein für eine Kryokonservierung geeigneter Behälter, beispielsweise ein Röhrchen, ein Straw (auch als Samenröhrchen bezeichnet), ein Beutel zur Blut- oder Stammzellenlagerung, eine Box oder ein anderer für eine Kryokonservierung geeigneter Behälter. Derartige Behälter werden entsprechend auch als Kryoröhrchen, Kryostraw, Kryobeutel, Kryobox oder allgemein als Kryobehälter bezeichnet.

Kryoröhrchen (engl. cryogenic tubes) werden auch als Biobank- oder Kryobankröhrchen bezeichnet. Kryoröhrchen weisen einen Aufnahmeraum auf, der einen inneren Hohlraum zur Aufnahme einer biologischen Probe ausbildet. Das Kryoröhrchen weist ferner üblicherweise einen Deckel zum Verschließen des Aufnahmeraums auf. Der Deckel kann einen Eingriff aufweisen, über den der Deckel mit einem Werkzeug gedreht werden kann. Das Kryoröhrchen kann auch ein Bodenelement aufweisen, das eine Kennung, z. B. in Form eines maschinenlesbaren Codes, aufweist.

Als Indikatorsubstanz kann eine Substanz ausgewählt werden, deren Schmelztemperatur einer vorbestimmten Grenztemperatur, deren Überschreiten überwacht werden soll, entspricht. Die Indikatorsubstanz ist eine Flüssigkeit oder eine Mischung verschiedener Flüssigkeiten, deren Schmelzpunkt der gewünschten Grenztemperatur entspricht. Lediglich beispielhaft kann als Indikatorsubstanz eine Mischung aus Wasser (H₂O) und Ethanol (C₂H₆O), eine Mischung aus Wasser (H₂O) und Kaliumhydroxid (KOH) oder eine Mischung aus Wasser und einem Gefrierschutzmittel gewählt werden. Das Mischungsverhältnis wird dabei gemäß dem jeweiligen Schmelzdiagramm, das den Verlauf des Schmelzpunktes in Abhängigkeit vom Mischungsverhältnis angibt, so eingestellt, dass der Schmelzpunkt des Flüssigkeitsgemisches den gewünschten Wert, nämlich die zu überwachende Grenztemperatur, aufweist.

In einer bevorzugten Ausführungsform umfasst die Indikatorsubstanz mindestens einen Alkohol, welcher aus der Gruppe, die Octan-1-ol, Nonan-1-ol, Propan-1,2-diol, Propan-1,3-diol, Butan-1,2-diol, Butan-1,3-diol, Butan-2-ol, Pentan-1,5-diol, Pentan-1-ol, Cyclopentanol, Benzylalkohol umfasst, ausgewählt ist. Besonders bevorzugt ist der mindestens eine Alkohol aus Propan-1,3-diol, Propan-1,2-diol und Butan-2-ol ausgewählt.

In einer anderen bevorzugten Ausführungsform umfasst die Indikatorsubstanz mindestens zwei verschiedene Alkoholkomponenten:
a) einen Alkohol, ausgewählt aus der Gruppe, die Octan-1-ol, Nonan-1-ol, Propan-1,2-diol, Propan-1,3-diol, Butan-1,2-diol, Butan-1,3-diol, Butan-2-ol, Pentan-1,5-diol, Pentan-1-ol, Cyclopentanol, Benzylalkohol umfasst;
b) einen Alkohol, ausgewählt aus der Gruppe, die Octan-l-ol, Nonan-l-ol, Propan-1,2-diol, Propan-1,3-diol, Butan-1,2-diol, Butan-1,3-diol, Butan-2-ol, Pentan-1,5-diol, Pentan-l-ol, Cyclopentanol, Benzylalkohol umfasst, mit einem niedrigeren Schmelzpunkt als der Alkohol der Komponente a);
wobei das Mischungsverhältnis der Komponenten a) und b) so eingestellt ist, dass die Schmelztemperatur der Mischung innerhalb eines Temperaturbereichs von -20 °C bis -160 °C, insbesondere von -25 °C bis -160 °C oder-50 °C bis-150 °C, liegt.

Speziellere Ausführungsformen sind dadurch gekennzeichnet, dass die Indikatorsubstanz eine der folgenden Kombinationen der Komponenten a) und b) umfasst:
- Octan-l-ol und Butan-2-ol in einem Mischungsverhältnis von 5 bis 95 Vol.-%;
- Octan-l-ol und Pentan-l-ol in einem Mischungsverhältnis von 5 bis 95 Vol.-%;
- Octan-l-ol und Propan-1,2-diol in einem Mischungsverhältnis von 5 bis 95 Vol.-%;
- Nonan-l-ol und Butan-2-ol in einem Mischungsverhältnis von 5 bis 95 Vol.-%;
- Nonan-l-ol und Propan-1,2-diol in einem Mischungsverhältnis von 5 bis 95 Vol.-%;
- Nonan-l-ol und Pentan-l-ol in einem Mischungsverhältnis von 5 bis 95 Vol.-%;
- Propan-1,2-diol und Butan-2-ol in einem Mischungsverhältnis von 5 bis 95 Vol.-%;
- Propan-1,2-diol und Propan-1,3-diol in einem Mischungsverhältnis von 5 bis 95 Vol.-%;
- Propan-1,2-diol und Butan-1,2-diol in einem Mischungsverhältnis von 5 bis 95 Vol.-%;
- Propan-1,3-diol und Butan-2-ol in einem Mischungsverhältnis von 5 bis 95 Vol.-%;
- Propan-1,3-diol und Butan-1,2-diol in einem Mischungsverhältnis von 5 bis 95 Vol.-%;
- Pentan-1,5-diol und Butan-2-ol in einem Mischungsverhältnis von 5 bis 95 Vol.-%;
- Benzylalkohol und Butan-2-ol in einem Mischungsverhältnis von 5 bis 95 Vol.-%;
- Pentan-l-ol und Butan-2-ol in einem Mischungsverhältnis von 5 bis 95 Vol.-%;
- Pentan-l-ol und Methanol in einem Mischungsverhältnis von 5 bis 95 Vol.-%;
- Cyclopentanol und Butan-2-ol in einem Mischungsverhältnis von 5 bis 95 Vol.-%;
- Cyclopentanol und Propan-1,2-diol in einem Mischungsverhältnis von 5 bis 95 Vol.-%;
- Cyclopentanol und Pentan-l-ol in einem Mischungsverhältnis von 5 bis 95 Vol.-%;
- Cyclopentanol und Butan-1,2-diol in einem Mischungsverhältnis von 5 bis 95 Vol.-%;
wobei der angegebene Wert des Mischungsverhältnisses sich jeweils auf den Anteil der erstgenannten Komponente in der Mischung aus beiden Komponenten bezieht.

In besonders bevorzugten Ausführungsformen umfasst diese Indikatormischung beispielsweise Propan-1,2-diol und Butan-2-ol in einem Mischungsverhältnis von 40 bis 60 Vol.-% (ergibt eine Schmelztemperatur von ca. - 90 °C), Propan-1,2-diol und Propan-1,3-diol in einem Mischungsverhältnis von 30 bis 70 Vol.-%, oder Propan-1,3-diol und Butan-2-ol in einem Mischungsverhältnis von 30 bis 70 Vol.-%.

Vorzugsweise umfasst die Indikatorsubstanz neben dem mindestens einen Alkohol noch mindestens einen Farbstoff wie oben beschrieben. Besonders bevorzugt ist dieser Farbstoff aus der Gruppe ausgewählt, welche Oil Red, Methylrot, Brillantgrün und Rhodamin B umfasst.

Eine noch speziellere Ausführungsform ist dadurch gekennzeichnet, dass die Indikator substanz mindestens zwei Alkohole a) und b), die aus Propan-1,3-diol, Propan-1,2-diol und Butan-2-ol ausgewählt sind, vorzugsweise in einem Mischungsverhältnis wie oben angegeben, sowie einen Farbstoff, der aus Gruppe ausgewählt ist, welche aus Oil Red, Methylrot, Brillantgrün und Rhodamin B besteht, umfasst.

Die Konzentration des Farbstoffs in der Alkoholkomponente kann je nach Farbstoff und Alkohol stark variieren.

In der Regel soll die Konzentration bei intensiver Färbung so niedrig wie möglich gehalten werden, damit die Farbmoleküle das Gefrier- und Schmelzverhalten der Alkohole, in denen sie gelöst werden, nicht verändern oder deren Viskosität erhöhen. Die Farbstoffkonzentration liegt dabei typischerweise in einem Bereich von < 10 Volumen-%, insbesondere < 1 % oder < 0,1 %, also im Prozent- oder Promille- bzw. Subpromillebereich.

In einer Variante der vorliegenden Erfindung entspricht die zu überwachende Grenztemperatur nicht direkt der Schmelztemperatur der Indikatorsubstanz, sondern vielmehr diejenigen Temperatur oberhalb der Schmelztemperatur, bei der die Viskosität der geschmolzenen Substanz soweit abgenommen hat, dass der erforderliche Flüssigkeitstransport stattfinden kann.

Diese Temperatur wird hier auch als Schwellentemperatur bezeichnet und liegt typischerweise in einem Temperaturbereich von 3-30 °C oder 5-30°C, beispielsweise 3-10 °C, 3-20 °C, 5-10 °C oder 5-20 °C, oberhalb der nominellen Schmelztemperatur.

In einer vorteilhaften Ausführungsform ist die Indikatorsubstanz daher dadurch gekennzeichnet, dass die flüssige Mischung in einem Temperaturbereich von 3-30 °C oder 5-30 °C oberhalb der Schmelztemperatur eine Viskosität in einem Bereich von 10 bis 10⁶ mPa*s, vorzugsweise 10 bis 10⁴ mPa*s, aufweist.

Gemäß einer bevorzugten Ausführungsform der Erfindung kann die Barriere ein in dem zweiten Teilbereich angeordnetes und an den ersten Teilbereich angrenzendes Material mit flüssigkeitsaufnehmender Struktur sein. Das Material kann ein poröses Material oder Medium sein. Das Material, z. B. eine Saugmasse, kann beispielsweise Filterpapier, z. B. wie das einer herkömmlichen Küchenrolle oder eines Zigarettenfilterpapiers, ein Presskörper, Zellulosescheiben, z. B. Taschentuchzellulosescheiben, Gips und/oder Kreidestaub, eine poröse Membran, ein Gewebe oder Gewirk, eine nano- oder mikroporöse Aluminiumoxidschicht sein. Dieses kann in den Eigenschaften so beeinflusst werden, z. B. durch Variation der porösen Struktur, dass es die Aufnahme der Flüssigkeit durch Kapillarkräfte befördert. Es kann jedoch auch ein anderweitig zur Flüssigkeitsaufnahme geeignetes Material sein. Gemäß dieser Ausführungsform liegt die Indikatorsubstanz anfangs im gefrorenen Zustand auf der Barriere in Form des Materials mit flüssigkeitsaufnehmender Struktur auf und kann nicht in dieses eindringen. Im flüssigen Aggregatszustand und somit nach Überschreiten der Schmelztemperatur der Indikatorsubstanz diffundiert die im ersten Teilbereich befindliche Indikatorsubstanz langsam in das an den ersten Teilbereich angrenzende Material mit flüssigkeitsaufnehmender Struktur hinein. Dadurch wird das Material von Indikatorsubstanz penetriert. Die Indikatorsubstanz gelangt auf diese Weise verzögert in den zweiten Teilbereich der Kammer. Wie stark die Verzögerung ist, hängt von der Diffusionsgeschwindigkeit ab.

Ein weiterer Vorteil ist, dass die Lage der Diffusionsfront ein Maß für die Zeit ist, die die Indikatorsubstanz und damit auch die Probe im Aufnahmeraum dem unerlaubten Temperaturbereich ausgesetzt war. Dies kann durch Feststellen der Lage der Diffusionsfront festgestellt werden. Zwar ist die Diffusionsgeschwindigkeit auch eine Funktion der Viskosität, die selbst temperaturabhängig ist; es genügt aber oft, eine Abschätzung zwischen wenigen Minuten und einigen Stunden bis zu Tagen vorzunehmen.

Somit ist es möglich, sowohl die Überschreitung einer Grenztemperatur als auch die Zeitdauer der Überschreitung zu erfassen. Das Grundprinzip dieser Ausführungsform besteht somit darin, in oder an dem Probenbehälter ein geschlossenes Volumen vorzusehen, in dem eine gefrorene Indikatorsubstanz sich auf einem Material befindet, in das diese langsam hineindiffundieren kann, wenn bei Überschreitung der Schmelztemperatur die bei der Lagertemperatur feste Indikatorsubstanz flüssig wird.

Ferner ist eine Kombinationen mehrerer Flüssigkeiten mit verschiedenen Schmelztemperaturen und mehreren Diffusionsstrecken, die sich vereinigen oder verzweigen, möglich, um so genauere Zeitangaben in Bezug auf die Dauer der Temperaturüberschreitung ermitteln zu können.

Gemäß Variante i) der Erfindung ist die Indikatorsubstanz in einem Behältnis gelagert das die Indikatorsubstanz im flüssigen Aggregatszustand dicht umschließt, zumindest solange des Behältnis noch nicht zerstört wurde, insbesondere zur Aktivierung der Temperaturüberwachung. Ferner weist die Vorrichtung zur Temperaturüberwachung ein in Bezug auf das Behältnis beweglich geführtes Aktivierungsteil auf, das von einer ersten Stellung, hier als Ausgangsstellung bezeichnet, in eine zweite Stellung, hier als Aktivierungsstellung bezeichnet, bringbar ist. Die Bewegung in die Aktivierungsstellung bewirkt, dass das Aktivierungsteil durch mechanischen Druck das Behältnis an mindestens einer Stelle zerstört, insbesondere derart, dass das Behältnis durchlässig für die Indikatorsubstanz im flüssigen Zustand wird. Mit anderen Worten wird somit ein Mechanismus bereitgestellt, mit dem die Vorrichtung zur Temperaturüberwachung zu einem gewünschten Zeitpunkt aktiviert bzw. scharfgeschaltet werden kann. Die Möglichkeit zur Aktivierung befördert den Handel und die Vorratshandlung, da die flüssige Indikatorsubstanz bis zur Aktivierung noch nicht in das angrenzende Material mit flüssigkeitsaufnehmender Struktur eindringen kann.

Das Behältnis kann als Plastikkissen ausgeführt sein, was besonders kostengünstig herstellbar ist. Das Behältnis kann als Glaskugel ausgeführt sein, was beim Zerstören ein hörbares Knack-Geräusch und damit ein akustisches Feedback der Aktivierung erzeugt.

Ferner kann der Probenbehälter einen Deckel zum Verschließen des Aufnahmeraums aufweisen, wobei die mindestens eine Kammer in den Deckel integriert sein kann. Hierbei kann der Deckel einen auf den Probenbehälter aufsteckbaren und/oder aufschraubbaren Grundkörper aufweisen, der im aufgeschraubten Zustand den Aufnahmeraum zur Aufnahme der biologischen Probe verschließt. Der Grundkörper kann einen H-förmigen Querschnitt aufweisen. Der Grundkörper kann zur Ausbildung der mindestens einen Kammer eine Ausnehmung aufweisen, in der das Behältnis mit der Indikatorsubstanz und das Material mit flüssigkeitsaufnehmender Struktur angeordnet sind. Vorstehend wurde erwähnt, dass die Vorrichtung zur Temperaturüberwachung ein in Bezug auf das Behältnis beweglich geführtes Aktivierungsteil aufweisen kann. Hierbei kann das Aktivierungsteil am Grundkörper beweglich in Richtung des Behältnisses geführt sein. Das Aktivierungsteil kann als Einschraubteil am Grundkörper angeordnet sein. Das Aktivierungsteil kann einen Vorsprung, der in Richtung zum Behältnis vom Aktivierungsteil abragt, aufweisen, z. B.in Form einer Spitze oder eines Dorns.

Eine weitere Möglichkeit der erfindungsgemäßen Realisierung sieht vor, dass die mindestens eine Kammer der Vorrichtung in einen Bodenbereich des Probenbehälters integriert ist. Hierbei kann ein Bodenbereich des Probenbehälters zur Ausbildung der mindestens einen Kammer eine Ausnehmung aufweisen, in der das Behältnis mit der Indikatorsubstanz und das Material mit flüssigkeitsaufnehmender Struktur angeordnet sind. Die Ausnehmung kann durch ein Bodenteil, in dem das Aktivierungsteil beweglich geführt ist, verschlossen werden. Das Bodenteil kann als Plastikkappe ausgeführt sein. Das Bodenteil kann vorzugsweise einen maschinenlesbaren und/oder optoelektronisch lesbaren Code, insbesondere einen Barcode, einen 2D-Code, einen 2D-Barcode und/oder einen QR-Code aufweisen.

Eine weitere Variante sieht vor, dass die Aktivierungsposition durch einen durch das Bodenteil gebildeten Anschlag festgelegt ist, bis zu dem das Aktivierungsteil in das Bodenteil hineingedrückt werden kann. Ferner kann das Bodenteil fest mit dem Bodenbereich des Probenbehälters verbunden sein, insbesondere verklebt, verschmolzen, verschweißt oder anderweitig fest mit dem Bodenbereich des Probenbehälters fixiert sein. Dadurch wird die Fälschungssicherheit der Temperaturüberwachung erhöht.

Bei einer vorteilhaften Variante, die nicht Teil der vorliegenden Erfindung bildet, weist eine an den ersten Teilbereich angrenzende Oberfläche des Materials eine Abdeckung auf. Die Abdeckung ist ausgelegt, bei Abkühlung der Vorrichtung auf eine Lagertemperatur, die unterhalb der Schmelztemperatur der Indikatorsubstanz liegt, von einem ersten Zustand der Abdeckung, in dem diese gegenüber der Indikatorsubstanz impermeabel ist, irreversibel in einen zweiten Zustand der Abdeckung, in dem diese für die Indikatorsubstanz permeabel ist, überzugehen. Im ersten Zustand ist die Abdeckung für die Indikatorsubstanz auch im flüssigen Aggregatszustand impermeabel, d. h. nicht durchlässig. Die Abdeckung bildet somit eine Trennschicht aus. Die Abdeckung kann beispielsweise eine entsprechend ausgelegte Membran, eine Abdeckschicht, eine Haut oder eine Folie oder Ähnliches sein, die erst durch das Schrumpfen bei Abkühlung auf die Lagertemperatur reißt oder anderweitig für Flüssigkeiten durchlässig wird, so dass die Indikatorsubstanz im flüssigen Aggregatszustand über die durch die mindestens eine Rissstelle oder allgemein Durchlassstelle gebildete Öffnung von dem ersten Teilbereich in den zweiten Teilbereich gelangen kann.

Dieses Konstruktionsprinzip hat den Vorteil, dass bereits mit Indikatorsubstanz befüllte Kammern vorgefertigt und verschlossen werden können. Diese können dann fertig konfektioniert gelagert und bei Bedarf mit der im Probenbehälter befindlichen Probe abgekühlt werden. Derartige als Indikatoreinrichtung dienende Kammern können daher fertig montiert und befüllt beliebig lange bei Raumtemperatur gelagert werden, da die Abdeckung dann nicht durchlässig ist.

Vorzugsweise wird für diese Abdeckung ein Material verwendet, das stärker bei Abnahme der Temperatur schrumpft als das Kammermaterial und dadurch Öffnungen bildet, durch die die flüssige Indikatorsubstanz in das Material mit flüssigkeitsaufnehmender Struktur eindringen kann. Bei ordnungsgemäßer Lagerung ist die Indikatorsubstanz gefroren und kann somit trotz der vorhandenen Öffnungen nicht in das Material oder in den zweiten Teilbereich der Kammer gelangen.

Gemäß einer weiteren Variante kann die Abdeckung, z. B. in Form einer Membran, durch mechanisches Verbiegen oder Eindrücken in den permeablen zweiten Zustand bringbar sein. So kann die Abdeckung erst kurz vor Nutzung durch Biegen oder Eindrücken für die Indikatorsubstanz im flüssigen Aggregatszustand permeabel gemacht werden.

Bei einer weiteren vorteilhaften Variante dieser Ausgestaltungsform können eine Struktur und/oder eine Zusammensetzung des Materials mit flüssigkeitsaufnehmender Struktur so ausgebildet sein, dass eine Diffusionsgeschwindigkeit der Indikatorsubstanz in dem Material nichtlinear mit zunehmenden Abstand vom ersten Teilbereich abnimmt.

Auf diese Weise lassen sich sehr kurze Aufenthaltszeiten (Sekunden bis zu Minuten) bei zu hoher Temperatur im oberen Bereich des Materials, d. h. dem Teil des Materials, der dem ersten Teilbereich zugewandt ist, und sehr lange Aufenthalte (Stunden bis Tage) im unteren Bereich, d. h. dem Teil des Materials, der dem ersten Teilbereich abgewandt ist, nachweisen.

vorteilhafte Ausführungsform gemäß Variante ii) der Erfindung sieht vor, dass die Barriere ein in Bezug auf die Indikatorsubstanz im flüssigen Zustand permeables Trennelement ist, das zwischen dem ersten und zweiten Teilbereich angeordnet ist. Die Barriere bildet somit eine Trennschicht zwischen erstem und zweitem Teilbereich aus, die für die Indikatorsubstanz nur im flüssigen, nicht jedoch im gefrorenen Zustand durchlässig ist. Das in Bezug auf die Indikatorsubstanz im flüssigen Zustand permeable Trennelement kann als poröse Trennwand, Membran, Folie, Haut oder Kapillarsystem ausgeführt sein.

Erfindungsgemäß ist hierbei vorgesehen, dass das Trennelement ausgelegt ist, bei Abkühlung der Vorrichtung auf eine Lagertemperatur, die unterhalb der Schmelztemperatur der Indikatorsubstanz liegt, aufgrund der thermischen Kontraktion an mindestens einer Stelle zu reißen, so dass die Indikatorsubstanz im flüssigen Aggregatszustand über die durch die mindestens eine Rissstelle gebildete Öffnung von dem ersten Teilbereich in den zweiten Teilbereich gelangen kann.

Diese Variante bietet wiederum den Vorteil, dass derartige als Indikatoreinrichtung dienende Kammern fertig montiert und befüllt beliebig lange bei Raumtemperatur gelagert werden können, da das Trennelement dann nicht durchlässig ist. Bei Bedarf kann die Kammer dann an dem Probenbehälter angeordnet und mit der im Probenbehälter befindlichen Probe abgekühlt werden, wobei sich dann die mindestens eine Rissstelle bildet. Bei ordnungsgemäßer Lagerung ist die Indikatorsubstanz gefroren und kann somit trotz der vorhandenen Öffnungen nicht in das Material oder in den zweiten Teilbereich der Kammer gelangen.

Das Trennelement kann mindestens eine Sollbruchstelle aufweisen, an der das Trennelement bei der Abkühlung der Vorrichtung auf die Lagertemperatur reißt. Eine Sollbruchstelle in diesem Sinne ist eine vorgegebene Stelle des Trennelements, an dem dieses bei der Abkühlung auf Lagertemperatur reißt. Dies bietet den Vorteil, dass die Lage und Größe der Rissstelle vorbestimmt ist und somit auch die dadurch ermöglichte Durchflussrate der Indikatorsubstanz, wenn diese flüssig ist.

Sollbruchstellen können beispielweise durch Ausdünnungen des Trennelements an bestimmten Stellen realisiert werden, beispielsweise in Form von punktförmigen Ausdünnungen am Rand der Trennschicht oder in Form von kreuzweise über die Trennschicht verlaufenden Linien. Derartige Trennschichten könnten beispielsweise im Spritzgussverfahren herstellt werden. Alternativ kann das Material auch so ausgelegt sein, dass es irregulär reißt.

Eine weitere vorteilhafte Variante der das Trennelement aufweisenden Ausführungsform sieht vor, dass in dem zweiten Teilbereich ein Gas vorhanden ist. Anstelle des Gases kann in dem zweiten Teilbereich auch eine Substanz vorhanden sein, die einen niedrigeren Schmelzpunkt als die Indikatorsubstanz aufweist. Die Substanz kann somit auch ein Stoff sein, der auch bei der Lagertemperatur oder zumindest vor dem Flüssigwerden der Indikatorsubstanz flüssig ist.

An der Veränderung einer Eigenschaft des Gases oder der Substanz, z. B. an dessen Farbe, Transparenz, optischen Drehwinkel, Impedanz etc., die aus der Vermischung mit der in den zweiten Teilbereich eintretenden Indikatorsubstanz resultiert, kann die Zeit der unberechtigten Temperaturerhöhung bestimmt werden. Da dieser Prozess nicht reversibel in dem angeschlossenen Volumen gestaltet ist, kann die so gebildete Vorrichtung zur Temperaturüberwachung als fälschungs- und manipulationssicher angesehen werden.

Ferner besteht im Rahmen der Erfindung die Möglichkeit, dass eine Außenwand der Kammer eine verschließbare Öffnung zum ersten Teilbereich aufweist, zum Befüllen des ersten Teilbereichs mit einer Indikatorsubstanz. Die Öffnung kann nach dem Befüllen mit der Indikatorsubstanz verschweißt, mit einer weiteren Substanz verschlossen oder anderweitig abgedichtet werden.

Die mindestens eine Kammer kann durch einen Behälter mit einem oder mehreren Hohlräumen gebildet sein, der außen am Probenbehälter anordenbar und/oder angeordnet ist. Der Begriff "anordenbar und/oder angeordnet" soll umfassen "befestigbar und/oder befestigt", "koppelbar und/oder gekoppelt", "verbindbar und/oder verbunden". Der Behälter zur Ausbildung der mindestens einen partiell mit Indikatorsubstanz gefüllten Kammer ist somit von dem Probenbehälter zu unterscheiden.

Der Behälter kann hierbei außen am Behälter anordenbar und/oder angeordnet sein oder im Inneren des Behälters. Eine Möglichkeit der erfindungsgemäßen Realisierung sieht vor, dass der Behälter zur Ausbildung der mindestens einen Kammer lösbar am Probenbehälter befestigt ist. Unter einer lösbaren Befestigung soll insbesondere auch ein Aufschieben oder Aufstecken des Behälters am Probenbehälter umfasst sein. Dies bietet den Vorteil, dass der Behälter räumlich getrennt vom Probenbehälter gelagert und vorbereitet (z. B. Befüllen mit der Indikatorsubstanz) werden kann.

Beispielsweise kann der Probenbehälter einen Deckel zum Verschließen des Aufnahmeraums aufweisen. Gemäß einem bevorzugten Ausführungsbeispiel kann die mindestens eine Kammer in den Deckel, z. B. in das Kopfteil und/oder den Schaft des Deckels, integriert sein. Beispielsweise kann der Deckel einen mit einem oberen Endbereich des Aufnahmeraums des Probenbehälters in Eingriff stehenden Schaft aufweisen, wobei die mindestens eine Kammer in den Schaft integriert ist.

Die Anordnung der mindestens einen Kammer im Deckel bietet den besonderen Vorzug, dass kein zusätzlicher Bauraum außerhalb des Probenbehälters vonnöten ist. Ein weiterer Vorteil ist, dass die als Indikatoreinrichtung dienende mindestens eine Kammer zusammen mit dem Deckel räumlich getrennt vom restlichen Probenbehälter gelagert und vorbereitet (z. B. Befüllen der Kammer mit der Indikatorsubstanz und Gefrieren der Indikatorsubstanz) werden kann. Besonders vorteilhaft ist eine Integration der Kammer in den Schaft des Deckels. Gemäß dieser Variante weist der Schaft des Deckels einen partiell mit Indikatorsubstanz gefüllten Hohlraum auf. Insbesondere bei Kryoröhrchen ist oftmals nur ein unteres Teilvolumen des Aufnahmeraums mit der Bioprobe befüllt, so dass ein oberes Teilvolumen zur Anordnung der Indikatorsubstanz genutzt werden kann.

Eine weitere Möglichkeit der erfindungsgemäßen Realisierung sieht vor, dass die mindestens eine Kammer durch einen Behälter gebildet ist und dass an einer Außenwand des Probenbehälters eine Aufnahme, beispielsweise eine Hülse oder Einstecktasche, befestigt ist, in die der Behälter zur Halterung am Probenbehälter einsteckbar und/oder eingesteckt ist.

Gemäß einer weiteren vorteilhaften Ausführungsform kann die mindestens eine Kammer durch ein doppelwandiges Aufsteckteil gebildet sein, das auf eine äußere Mantelfläche des Probenbehälters aufsteckbar oder aufschiebbar ist und diese im aufgesteckten Zustand dabei zumindest teilweise umgreift. Diese Variante ist besonders vorteilhaft für zylinderförmige Probenbehälter, insbesondere Kryoröhrchen. Das doppelwandige Aufsteckteil kann dabei als Hohlzylinder oder Teil-Hohlzylinder ausgeführt sein, dessen Innendurchmesser dem Außendurchmesser des Probenbehälters entspricht, so dass das Aufsteckteil den zylinderförmigen Probenbehälter manschettenartig oder schellenartig umgreift.

Der Probenbehälter kann ferner mit dem Aufsteckteil verklebt, verschmolzen oder anderweitig fest fixiert sein. Dadurch kann ein Entfernen des Aufsteckteils zu Manipulationszwecken, z. B. um ein Aufsteckteil, das eine unerwünschte Erwärmung anzeigt, durch ein neues Aufsteckteil zu ersetzen, verhindert werden.

Der Probenbehälter kann aber auch ein an sich bekannter Beutel zur Ablage und/oder Kryolagerung von Blutproben oder Stammzellen sein, der in einer Kassette gehaltert ist. Die mindestens eine Kammer kann durch einen Behälter gebildet sein, der an einer Außenseite des Beutels befestigt ist oder der an der Kassette befestigt ist. Die mindestens eine Kammer kann auch durch einen Behälter gebildet sein, der freischwimmend im Innern des Beutels vorhanden ist. Der Behälter mit der Indikatorsubstanz kann hierbei in der Dichte so kalibriert sein, dass er zentral in der Beutelflüssigkeit schwimmt und dort die Kerntemperatur des Beutels erfasst.

Gemäß einer weiteren bevorzugten Ausführungsform kann die Vorrichtung eine Mehrzahl von Kammern aufweisen, die jeweils lediglich zum Teil mit einer Indikatorsubstanz, deren Schmelztemperatur in einem Bereich von -20 °C bis -140 °C liegt, gefüllt sind, und eine Barriere aufweisen, wie vorstehend beschrieben wurde. Beispielsweise kann ein Behälter zur Ausbildung mehrerer Kammer mehrere durch Trennwände gebildete Teilhohlräume aufweisen. Hierbei bildet jeder Teilhohlraum eine Kammer aus, die die Indikatorsubstanz und die Barriere enthält. Die Indikatorsubstanzen in den Kammern können unterschiedliche Schmelztemperaturen aufweisen. Damit können unterschiedliche Temperaturgrenzwerte überwacht werden, wobei jede Indikatorsubstanz so ausgewählt und/oder deren Mischungsverhältnis so eingestellt ist, dass ihr Schmelzpunkt einem der zu überwachenden Temperaturgrenzwerte entspricht. Diese Ausführungsform bietet den Vorteil, dass sich die erreichten Temperaturintervalle, in die die Probe gelangt ist, genauer eingrenzen lassen.

Gemäß einer weiteren Ausführungsform der Erfindung kann die mindestens eine Kammer in den Probenbehälter selbst integriert sein, d. h., der Probenbehälter selbst kann in seinem Innern eine solche Kammer oder mehrere aufweisen. Dadurch kann auf ein separates außen am Probenbehälter angeordnetes Bauteil zur Ausbildung der mindestens einen Kammer verzichtet werden. Beispielsweise kann der Aufnahmeraum des Probenbehälters zur Ausbildung der mindestens einen Kammer doppelwandig mit einer Innenwandung und einer Außenwandung ausgeführt sein, wobei ein Zwischenraum zwischen der Innenwandung und der Außenwandung zum Teil mit der Indikatorsubstanz gefüllt ist. Zur Ausbildung mehrerer Kammern kann der Zwischenraum durch Trennwände in Teilräume unterteilt sein.

Gemäß einer bevorzugten Ausführungsform kann die Vorrichtung eine Messeinrichtung aufweisen, die ausgebildet ist, das Vorhandensein der Indikatorsubstanz im zweiten Teilbereich der Kammer und/oder einen Füllstand der Indikatorsubstanz in dem ersten und/oder dem zweiten Teilbereich der Kammer zu erfassen. Die Messeinrichtung kann lediglich beispielhaft eine optische oder optisch-elektrische Messeinrichtung sein, um z. B. mit einer optischen Transmissions-, Streulicht- oder Reflektionsmessung eine Konfigurationsänderung der Indikatorsubstanz festzustellen.

Gemäß einem zweiten Gesichtspunkt der Erfindung werden die genannten Aufgaben durch ein Verfahren zur Temperaturüberwachung von kryokonservierten Proben gelöst, das eine Vorrirhtung zur Temperaturüberwachung nach einem der Ansprüche 1-19 verwendet. Die Ausführungen betreffend die Vorrichtung, insbesondere deren vorteilhafte Ausführungsvarianten, sollen somit zur Vermeidung von Wiederholungen auch als verfahrensgemäß offenbart gelten

Als Indikatorsubstanz kann vorzugsweise wiederum eine Substanz ausgewählt werden, deren Schmelztemperatur einer vorbestimmten Grenztemperatur, deren Überschreiten überwacht werden soll, entspricht.

Gemäß dem Verfahren kann somit eine Vorrichtung zur Temperaturüberwachung bereitgestellt werden, wobei die Vorrichtung mindestens eine Indikatorsubstanz im gefrorenen Zustand im ersten Teilbereich der Kammer enthält. Der Aufnahmeraum des Probenbehälters enthält eine kryokonservierte biologische Probe. Das Verfahren umfasst ferner das gekühlte Lagern der Vorrichtung zur Kryokonservierung. Das Verfahren umfasst ferner das Prüfen, ob sich zu einem späteren Zeitpunkt im zweiten Teilbereich der Kammer Indikatorsubstanz befindet.

Ist dies der Fall, kann auf ein Überschreiten der Schmelztemperatur der Indikatorsubstanz und somit der zu überwachenden Grenztemperatur geschlossenen werden, insbesondere auch dann, wenn die Überschreitung nur kurzzeitig aufgetreten ist.

Ein besonderer Vorzug der Erfindung liegt somit darin, dass das Vorhandensein von Indikatorsubstanz im zweiten Teilbereich direkt anzeigt, dass eine Kryoprobe sich über eine definierbare Grenztemperatur, und wenn auch nur kurzzeitig, erwärmt hat. Dies kann durch visuelle Sichtprüfung oder auch technisch automatisiert mittels einer entsprechend eingerichteten Messeinrichtung schnell und einfach festgestellt werden, ohne dass die Probe aus dem Probenbehälter entnommen oder aufgetaut werden muss.

Gemäß einer vorteilhaften Weiterbildung des Verfahrens kann ferner eine Kenngröße bestimmt werden, die ein Maß der in den zweiten Teilbereich der Kammer übergegangenen Menge an Indikatorsubstanz und/oder ein Maß der im ersten Teilbereich der Kammer befindlichen Menge an Indikatorsubstanz angibt. Eine derartige Kenngröße gibt ein Maß für die Dauer an, die die Probe oberhalb der Grenztemperatur verbracht hat.

Eine Kenngröße ist beispielsweise eine Größe, die die Menge an Indikatorsubstanz im ersten oder im zweiten Teilbereich angibt. Die Kenngröße kann der Füllstand der Indikatorsubstanz im zweiten Teilbereich sein oder die Länge der Diffusionsstrecke, die die Indikatorsubstanz im Material mit der flüssigkeitsaufnehmenden Struktur erreicht hat. Gemäß einer besonders bevorzugten Ausführungsform des Verfahrens wird zur Durchführung des Verfahrens eine Vorrichtung zur Temperaturüberwachung verwendet, die in Trennelement aufweist, das erst beim Abkühlen durchlässig für die Indikatorsubstanz im flüssigen Aggregatszustand wird. Vorstehend wurde bereits beschrieben, dass zwischen dem ersten und zweiten Teilbereich ein Trennelement angeordnet sein kann, das ausgelegt ist, bei Abkühlung der Vorrichtung auf eine Lagertemperatur, die unterhalb der Schmelztemperatur der Indikatorsubstanz liegt, aufgrund der thermischen Kontraktion an mindestens einer Stelle zu reißen, so dass die Indikatorsubstanz im flüssigen Aggregatszustand über die durch die mindestens eine Rissstelle gebildete Öffnung von dem ersten Teilbereich in den zweiten Teilbereich gelangen kann.

Ebenfalls wurde beschrieben, dass in einer Ausführungsform, die nicht Teil der vorliegenden Erfindung bildet, eine an den ersten Teilbereich angrenzende Oberfläche des Materials eine Abdeckung, z. B. eine Membran oder Abdeckschicht, aufweisen kann, die ausgelegt ist, bei Abkühlung der Vorrichtung auf eine Lagertemperatur, die unterhalb der Schmelztemperatur der Indikatorsubstanz liegt, von einem ersten Zustand der Abdeckung, in dem diese gegenüber der Indikatorsubstanz impermeabel ist, irreversibel in einen zweiten Zustand der Abdeckung, in dem diese für die Indikatorsubstanz permeabel ist, überzugehen.

Gemäß der besonders bevorzugten Ausführungsform des Verfahrens kann zur Überprüfung der Funktionstüchtigkeit der Vorrichtung zur Temperaturüberwachung, insbesondere der Funktionsfähigkeit der Indikatoreinrichtung, zumindest einer der folgenden Prüfschritte durchgeführt werden:
Erstens kann geprüft werden, ob der zweite Teilbereich der mindestens einen Kammer nach dem Befüllen des ersten Teilbereichs mit Indikatorsubstanz und vor dem Einfrieren der Indikatorsubstanz frei von Indikatorsubstanz ist. In diesem Zustand müsste das als Barriere dienende Trennelement noch intakt sein, so dass keine Indikatorsubstanz im unter dem ersten Teilbereich befindlichen zweiten Teilbereich vorhanden sein dürfte. Ist dies doch der Fall, kann die Kammer nicht benutzt werden, da diese bereits defekt ist. Gleiches gilt für den Fall, falls statt einer Kammer mit Trennelement eine Kammer mit einer Abdeckung verwendet wird.

Zweitens kann geprüft werden, ob der zweite Teilbereich der mindestens einen Kammer nach dem Gefrieren der im ersten Teilbereich der mindestens einen Kammer befindlichen Indikatorsubstanz und vor dem gekühlten Lagern der Vorrichtung zur Kryokonservierung frei von Indikatorsubstanz ist. Nach dem Abkühlen der Vorrichtung auf Lagertemperatur oder zumindest unterhalb der Schmelztemperatur der Indikatorsubstanz und der Temperatur, an der das Trennelement reißen sollte, sollte das Trennelement gerissen und die Indikatorsubstanz gefroren sein, so dass wiederum keine Indikatorsubstanz im unteren zweiten Teilbereich sein sollte. Ist das nicht der Fall, ist eine neue Kammer zu verwenden. Gleiches gilt wiederum für den Fall, dass statt einer Kammer mit Trennelement eine Kammer mit einer Abdeckung verwendet wird.

Drittens kann geprüft werden, falls eine kryokonservierte Probe zur Nutzung entnommen wird und falls sich im zweiten Teilbereich der Kammer dann keine Indikatorsubstanz befindet, ob die Abdeckung ordnungsgemäß in den permeablen zweiten Zustand übergegangen ist oder ob das Trennelement oder die Abdeckung der mindestens einen Kammer ordnungsgemäß gerissen ist. Es könnte nämlich der Fall sein, dass das Trennelement oder die Abdeckung nicht gerissen ist und damit die Überschreitung der Schmelztemperatur der Indikatorsubstanz nicht angezeigt wurde. Dies kann beispielsweise geprüft werden, indem die betreffende Kammer mit Indikatorsubstanz nach dem Auftauen eine gewisse Zeit liegengelassen wird. Die Probe kann währenddessen schon weiterverarbeitet werden. War das Trennelement oder die Abdeckung ordnungsgemäß gerissen und die Lagertemperatur lag die ganze Zeit unterhalb der Schmelztemperatur der Indikatorsubstanz, dann wird nun bei Raumtemperatur in jedem Fall die Indikatorflüssigkeit durch die Barriere in den zweiten Teilbereich gelangen.

Vorzugsweise werden alle diese Prüfschritte im Rahmen einer Temperaturüberwachung einer kryokonservierten Probe durchgeführt. Auf diese Weise kann die Funktionsfähigkeit der Kammer bewiesen und eindeutig belegt werden, dass die Lagertemperatur über die gesamte Zeit den Vorgaben entsprochen hat.

Mit dem Begriff Probenbehälter wird insbesondere ein für eine Kryokonservierung ausgelegter Behälter bezeichnet. Der Probenbehälter ist vorzugsweise unter Verwendung tieftemperaturverträglichen Kunststoffmaterials für Temperaturen unter -140 °C hergestellt. Das Kunststoffmaterial kann ohne Veränderung und ohne Schaden wiederholte Temperaturwechsel tolerieren. Es wird vorzugsweise ein Kunststoffmaterial verwendet, dessen Wasseraufnahmefähigkeit < 1 % der Eigenmasse, insbesondere < 0.1 % der Eigenmasse beträgt. Erfindungsgemäße Kryospeicherelemente basieren beispielsweise auf Polyurethan oder Polyethylen.

Mit dem Begriff "biologische Probe", in diesem Dokument auch kurz als Probe bezeichnet, wird biologisches Material wie Zellen, Gewebe, Zellbestandteile, biologische Makromoleküle etc. bezeichnet, welches im Probenbehälter der Kryokonservierung unterzogen wird - ggf. in einer Suspension und/oder im Verbund mit einem Substratmaterial. Im Aufnahmeraum kann somit ein Substrat angeordnet sein, das zur adhärenten Aufnahme biologischer Zellen, die Teil der biologischen Probe sind, eingerichtet ist.

Die zuvor beschriebenen bevorzugten Ausführungsformen und Merkmale der Erfindung sind miteinander kombinierbar. Weitere Einzelheiten und Vorteile der Erfindung werden im Folgenden unter Bezug auf die beigefügten Zeichnungen beschrieben. Es zeigen:
- FIG. 1-4: schematische Ansichten verschiedener Ausführungsbeispiele eine Vorrichtung zur Temperaturüberwachung einer kryokonservierten biologischen Probe;
- FIG. 5: schematisch verschiedene Ausführungsvarianten einer Kammer mit einem Trennelement als Barriere;
- FIG. 6: ein Ablaufdiagramm zur Illustration eines Ausführungsbeispiels eines Verfahrens zur Temperaturüberwachung einer kryokonservierten biologischen Probe;
- FIG. 7A, 7B, 8A: jeweils ein Schmelzdiagramm eines Flüssigkeitsgemisches;
- FIG. 8B: eine Tabelle mit Schmelzpunkten einiger reiner Flüssigkeiten;
- FIG. 9: eine Mischbarkeitsmatrix von Lösemitteln;
- FIG. 10: ein Ausführungsbeispiel einer Vorrichtung zur Temperaturüberwachung;
- FIG. 11: ein Ausführungsbeispiel einer Vorrichtung zur Temperaturüberwachung, und
- FIG. 12: ein Ausführungsbeispiel einer Vorrichtung zur Temperaturüberwachung.

Gleiche oder funktional äquivalente Elemente sind in allen Figuren mit denselben Bezugszeichen bezeichnet und werden zum Teil nicht gesondert beschrieben.

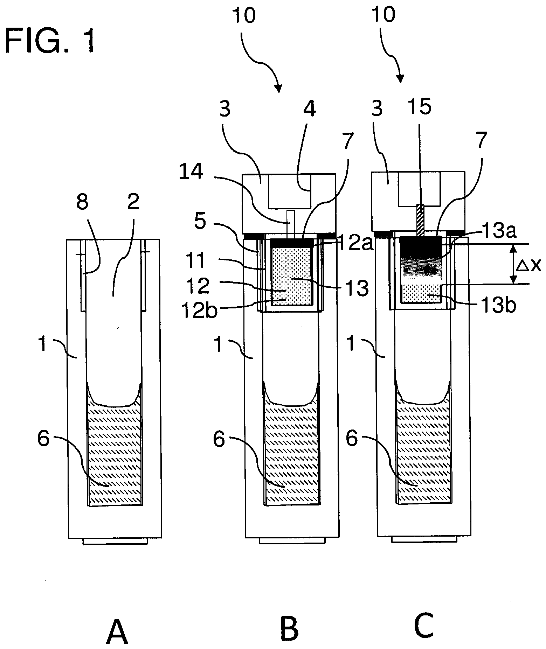

Die in den Figuren 1-4 gezeigten Ausführungsformen sind nicht erfindungsgemäß und dienen lediglich der Veranschaulichung.

Gezeigt ist in Figur 1A beispielhaft ein Kryoröhrchen 1, stellvertretend für andere Kryoprobenbehälter, wie Straws, Beutel, Boxen etc.

Das Kryoröhrchen umfasst ein Aufnahmevolumen 2 für die Bioprobe, in dem sich bei Verwendung der Vorrichtung die Biomaterialien befinden. Die Bioprobe ist hier eine Zellsuspension 6. Das Kryoröhrchen ist in Figur 1A noch ohne Schraubdeckelverschluss gezeigt. Das Kryoröhrchen umfasst einen in Figur 1B gezeigten Deckel 3, der das Gefäß verschließt und oben einen Eingriff 4 besitzt, über den der Deckel 3 mit einem Werkzeug (nicht gezeigt) im Fall der Automatisierung gedreht werden kann. Diese Kryoröhrchen 1 können auch einen Boden enthalten, in den optional ein Barcodeviereck oder eine andere Kennung eingefügt ist. In dieser Form, zumeist senkrecht in Aufnahmen stehend, werden die Kryoröhrchen 1 in den Tieftemperaturbehältern gelagert.

In Figur 1B ist das lagerbereite System dargestellt. Der Deckel hat ein auf dem Aufnahmevolumen 2 aufsitzendes Kopfteil und einen daran angeformten Schaft 5, der in das Aufnahmevolumen 2 im verschraubten Zustand eingreift. Der korrespondierende Bereich des Aufnahmevolumens 2 weist ein Gewinde 8 auf. In den Schraubdeckel 3 ist eine Kammer 11 integriert, die ein in sich abgeschlossenes Volumen 12 ausbildet. Das Innenvolumen 12 der Kammer 11 ist in Figur 1B durch den schwarzen und gepunkteten Bereich dargestellt. In diesem Volumen 12 befindet sich in einem zweiten Teilbereich 12b ein poröses oder anderweitig zur Aufnahme einer Flüssigkeit geeignetes Material 13 und darüber ein kleiner Hohlraum (erster Teilbereich) 12a, der über eine kleine Öffnung 14 mit der Indikatorsubstanz 7 befüllt wird. Die Indikatorsubstanz 7 wird bei Unterschreiten der gewünschten Grenztemperatur, z. B. -70 °C, fest und bei Überschreiten wieder flüssig.

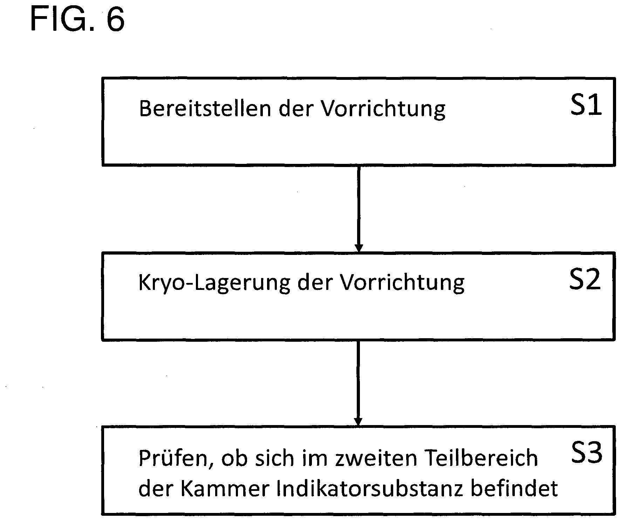

Über die Auswahl geeigneter Flüssigkeiten und das Mischungsverhältnis von Flüssigkeiten kann deren Schmelzpunkt auf einen gewünschten Wert in einem Bereich von -20 °C bis -140 °C festgelegt werden und so eine geeignete Indikatorsubstanz 7 je nach zu überwachender Grenztemperatur ausgewählt werden, was nachfolgend noch in Zusammenhang mit den Figuren 6 bis 9 näher erläutert wird.

Das Befüllen des ersten Teilbereichs 12a erfolgt zweckmäßigerweise bei der Abkühlung, kurz vor dem Einstellen der Lagertemperatur des Kryoröhrchens 1, d. h. in der Regel unterhalb von -140 °C, so dass die Indikatorsubstanz 7 nicht genügend Zeit hat, in das Material 13 hinein zu penetrieren, was je nach Aufbau Minuten, Stunden oder Tage betragen kann.

Bei der Lagertemperatur ist die Indikatorsubstanz 7 dann fest, so dass sich an der Anordnung, wie sie in Figur 1B dargestellt ist, nichts mehr ändert. Die Öffnung 14 wird verschweißt, mit einer Substanz 15 verschlossen oder anderweitig abgedichtet.

Die so gebildete Vorrichtung 10 aus Probenbehälter 1 mit integrierter Kammer 11 ist zur Temperaturüberwachung einer kryokonservierten biologischen Probe 6 ausgelegt. Figur 1B zeigt die Vorrichtung in einem Anfangszustand und in einem Zustand, wenn keine zwischenzeitliche Überschreitung der Schmelztemperatur der Indikatorsubstanz 7 stattgefunden hat. Figur 1C zeigt die Vorrichtung in einem Zustand, falls eine zwischenzeitliche Überschreitung der Schmelztemperatur der Indikatorsubstanz 7 stattgefunden hat.

Ist die Probe 6 nun zu irgendeinem Zeitpunkt über die Schmelztemperatur der Indikatorsubstanz 7, beispielsweise ein gefärbtes Alkohol-Wassergemisch mit einer Schmelztemperatur von -70 °C, erwärmt worden, dann dringt die nun flüssige Indikatorsubstanz 7 in das Material 13 ein, wodurch sich ein Zustand der Vorrichtung 10, wie in Figur 1C dargestellt, ergibt. Figur 1C zeigt einen Zustand, in dem die Indikatorsubstanz bereits in einen oberen Bereich 13a der Länge Δx des Materials 13 hineindiffundiert ist, aber den unteren Bereich 13b noch nicht erreicht hat.

Die Wegstrecke (Diffusionsstrecke) Δx ist ein Maß für die Dauer, die die Probe 6 oberhalb der Grenztemperatur verbracht hat. Da dieser Prozess nicht rückgängig gemacht werden kann, ist das System fälschungssicher, solange nicht der gesamte Schraubverschluss 3 ausgetauscht wird, was allerdings über Markierungen, Codes etc. erfasst und damit verhindert werden kann.

Der Aufbau einer solchen Kammer 11 kann nun in vielfältiger Weise variiert werden. So kann einmal das poröse Material 13 mit einer Abdeckung (nicht dargestellt), z. B. einer dünnen Haut oder Membran, bedeckt sein, die erst durch das Schrumpfen bei Abkühlung auf die Lagertemperatur reißt oder anderweitig für die Indikatorsubstanz 7 im flüssigen Aggregatszustand durchlässig wird. Dieses Konstruktionsprinzip hat den Vorteil, dass bereits befüllte Deckel vorgefertigt, verschlossen und fertig konfektioniert gelagert werden können und wie die bisher üblichen Deckel bei Benutzung nur aufgeschraubt und mit der Probe 6 und dem Probenbehälter 1 abgekühlt werden können.

Geeigneterweise kann für diese Abdeckung ein Material verwendet werden, das stärker bei Abnahme der Temperatur schrumpft als das Deckelmaterial und dadurch Öffnungen bildet, durch die die Indikatorsubstanz 7 im flüssigen Aggregatszustand in das Material 13 eindringen kann. Bei ordnungsgemäßer Lagerung ist die Indikatorsubstanz 7 gefroren und kann somit trotz der vorhandenen Öffnungen nicht in das Material 13 oder in den zweiten Teilbereich 12b gelangen.

Variationsmöglichkeiten ergeben sich des Weiteren über die Struktur und den Aufbau des Materials 13. Dieses kann in den Eigenschaften so beeinflusst werden, dass es die Aufnahme der Flüssigkeit durch Kapillarkräfte befördert oder auch eine erwünschte Diffusionsgeschwindigkeit eingestellt wird, die nichtlinear mit dem Abstand, aber in bekannter Weise nach unten abnimmt. Auf diese Weise lassen sich sehr kurze Aufenthaltszeiten (Sekunden bis zu Minuten) bei zu hoher Temperatur im oberen Bereich und sehr lange Aufenthalte (Stunden bis Tage) im unteren Bereich des Materials 13 nachweisen.

Es kann auch eine Zwischenschicht als Abdeckung verwendet werden, die kurz vor der Nutzung durch Biegen oder Eindrücken von außen permeabel gemacht wird.

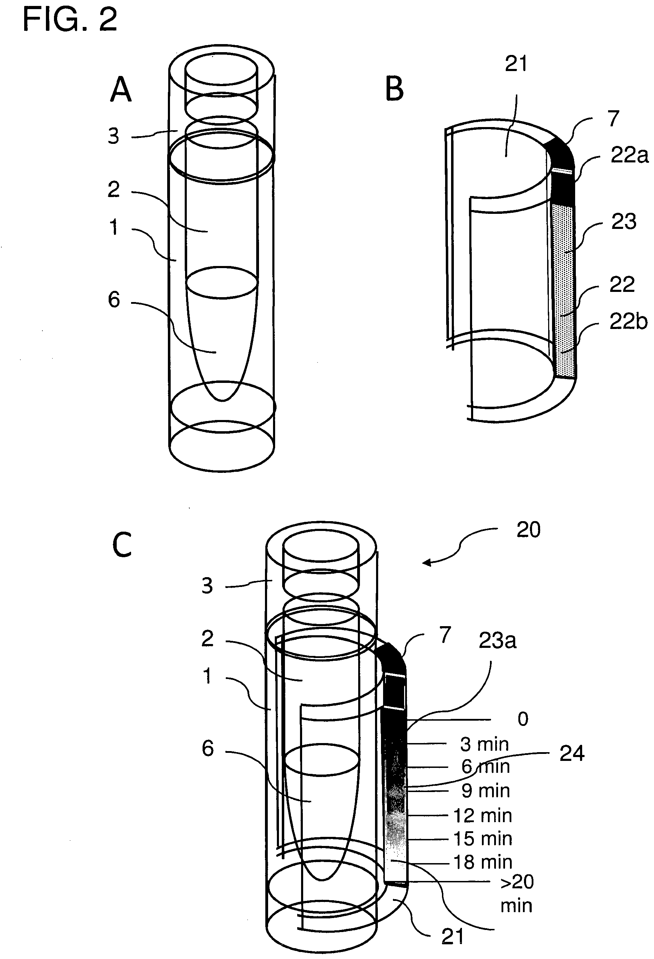

Die Ansichten A, B und C der Figur 2 illustrieren eine weitere Ausgestaltungsform wobei Figur 2C eine Vorrichtung 20 zur Temperaturüberwachung einer kryokonservierten biologischen Probe in einem Zustand zeigt, nachdem eine zwischenzeitliche Überschreitung der Schmelztemperatur der Indikatorsubstanz 7 stattgefunden hat.

Figur 2A zeigt zunächst ein mit einem Deckel 3 komplett verschlossenes Kryoröhrchen 1, wie es üblicherweise in Kryobanken verwendet wird. Figur 2B zeigt ein doppelwandig ausgeführtes Aufsteckteil 21 aus Plastik, das auf die äußere Mantelfläche des Kryoröhrchens 1 aufgesetzt werden kann, wie in Figur 2C dargestellt.

In diesem Plastikteil 21 befindet/befinden sich analog zu dem in Figur 1 illustrierten Ausführungsbeispiel in senkrechter Ausrichtung ein oder mehrere Volumina 22, in denen sich jeweils in einem oberen Teilbereich 22a eine Indikatorsubstanz 7 im gefrorenen Zustand auf einem porösen Medium 23 befindet. Das poröse Medium befindet sich in dem unteren Teilbereich 22b des Volumens 22. Das Plastikteil 21 kann somit eine erfindungsgemäße Kammer ausbilden oder auch mehrere, falls das Innenvolumen 22 des Plastikteils durch Trennwände in mehrere Teilvolumina unterteilt ist, die jeweils eine Indikatorsubstanz und ein poröses Medium aufweisen.

Analog zu dem in Figur 1 illustrierten Ausführungsbeispiel diffundiert die Indikatorsubstanz 7 erst dann in das darunter befindliche Material 13 hinein, wenn eine Schmelztemperatur der Indikatorsubstanz 7 überschrittet wurde und die Indikatorsubstanz 7 schmilzt.

Figur 2C zeigt einen Zustand, bei dem die Indikatorsubstanz bereits in einen oberen Bereich 23a des porösen Mediums 23 hineindiffundiert ist.

Die Diffusionsstrecke liegt bei dieser Ausführungsform bei wenigen Millimetern bis zu mehr als zehn Zentimetern, je nach Größe und Geometrie des Probenbehälters 1. Das Plastikteil 21 kann mit einer Skala 24 versehen sein, die eine genauere Erfassung der Zeiten erlaubt.

Alternative Ausführungsformen sind des Weiteren Hülsen oder Zylinderteile, die in ähnlicher Weise auf das Kryoröhrchen 1 geschoben werden können. Zur Fälschungssicherheit kann das Aufsatzteil verklebt, angeschmolzen oder anderweitig fest fixiert werden.

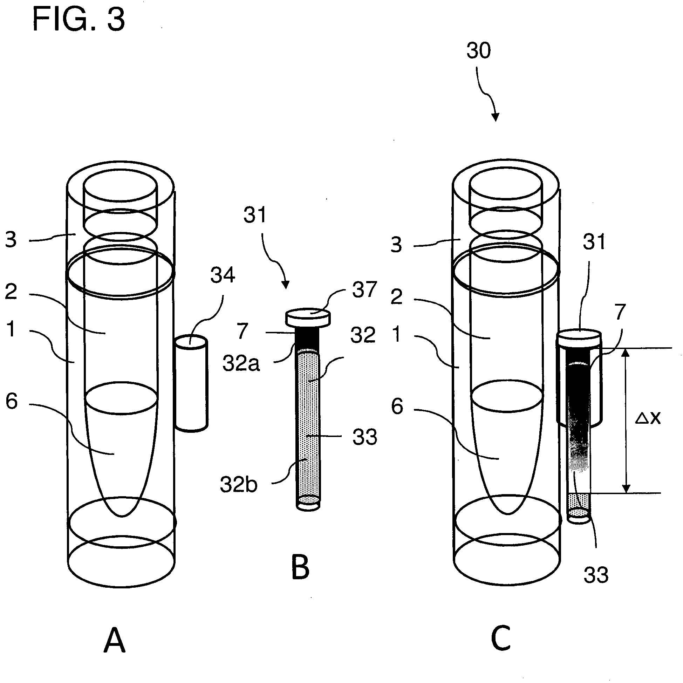

Die Ansichten A, B und C der Figur 3 illustrieren eine weitere Ausgestaltungsform wobei Figur 3C eine Vorrichtung 30 zur Temperaturüberwachung einer kryokonservierten biologischen Probe in einem Zustand zeigt, nachdem eine zwischenzeitliche Überschreitung der Schmelztemperatur der Indikatorsubstanz 7 stattgefunden hat.

Figur 3A zeigt wieder ein mit einem Deckel 3 komplett verschlossenes Kryoröhrchen 1. Im Unterschied zu dem in Figur 2A gezeigten Kryoröhrchen ist an einer seitlichen Außenfläche des Kryoröhrchens 1 eine Aufnahme 34 befestigt, in die ein in Figur 3B dargestellter Behälter 31 eingeschoben wird. Dieser Behälter 31 bildet ein zylindrisches oder anders geformtes Innenvolumen 32 aus. Der Behälter ist gegen Herausfallen aus der Aufnahme 34 über eine Scheibe 37 gesichert. In einem ersten Teilbereich 32a des Innenvolumens 32 befindet sich die Indikatorsubstanz 7, optional auf einer Trennschicht (nicht dargestellt), die erst beim Abkühlen auf Lagertemperatur für die Indikatorsubstanz im flüssigen Aggregatszustand durchlässig wird, und darunter in einem zweiten Teilbereich 32b das poröse Aufnahmematerial 33. Bei einem porösen Material mit starker Kapillarwirkung spielt die Lage der Einheit eine untergeordnete Rolle. Eine senkrechte Anordnung und Lagerung der Probe ist jedoch empfehlenswert.

Figur 3C zeigt die aus Kryoröhrchen und Behälter 31 gebildete Vorrichtung 30 zur Temperaturüberwachung in einem Zustand, nachdem eine zwischenzeitliche Überschreitung der Schmelztemperatur der Indikatorsubstanz 7 stattgefunden hat. Es ist erkennbar, dass im Vergleich zum in Figur 3B gezeigten Zustand des Behälters die Indikatorsubstanz in das poröse Material 33 hineindiffundiert ist. Δx gibt wieder die Länge der Diffusionsstrecke an.

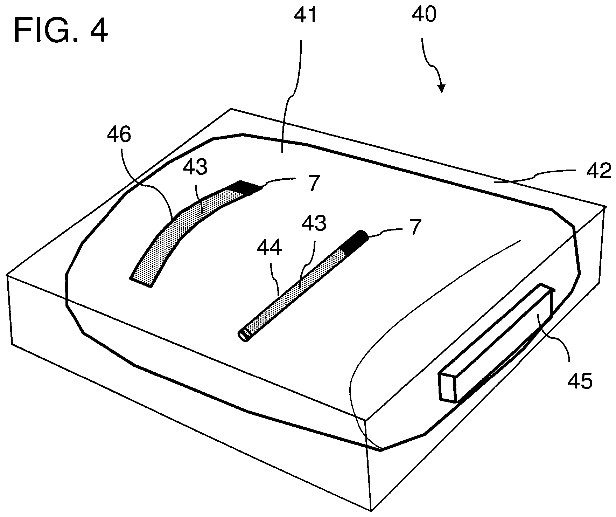

Figur 4 zeigt einen Beutel 41, wie er zur Ablage von Stammzellen und Blutproben Anwendung findet. Häufig befinden sich diese Beutel noch in einer Kassette 42, die aus Aluminium besteht. Eine Vorrichtung 40 zur Temperaturüberwachung einer kryokonservierten biologischen Probe kann wiederum aus dem Probenbehälter in Form des Beutels 41 und einem temperatursensitiven System gebildet werden. Das temperatursensitive System wird analog zu den Kammern bzw. Behältern 11, 21, 31 der Figuren 1 bis 3 wiederum durch einen Behälter 44, 45 oder 46 gebildet, der lediglich zum Teil mit einer Indikatorsubstanz 7 gefüllt ist. Der Behälter weist ferner ein poröses Material 43 auf, das bewirkt, dass, wenn sich Indikatorsubstanz 7 in einem ersten Teilbereich der Kammer im flüssigen Aggregatszustand befindet, diese Indikatorsubstanz 7 verzögert in einen zweiten Teilbereich des Behälters gelangt.

Der Behälter 43 kann nun auf dem Beutel 41 an einer Außenseite des Beutels angebracht werden. Der Behälter 45 kann auch auf der Kassette 42 außen oder innen angebracht werden. Der Behälter 44 kann auch frei schwimmend oder fixiert im Inneren des Beutels 41 angebracht werden. Die Variante innen erfordert Sterilisierung und inerte Materialien außen. Dieses System kann in der Dichte so kalibriert werden, dass es zentral in der Beutelflüssigkeit schwimmt und dort die Kerntemperatur des Beutels erfasst.

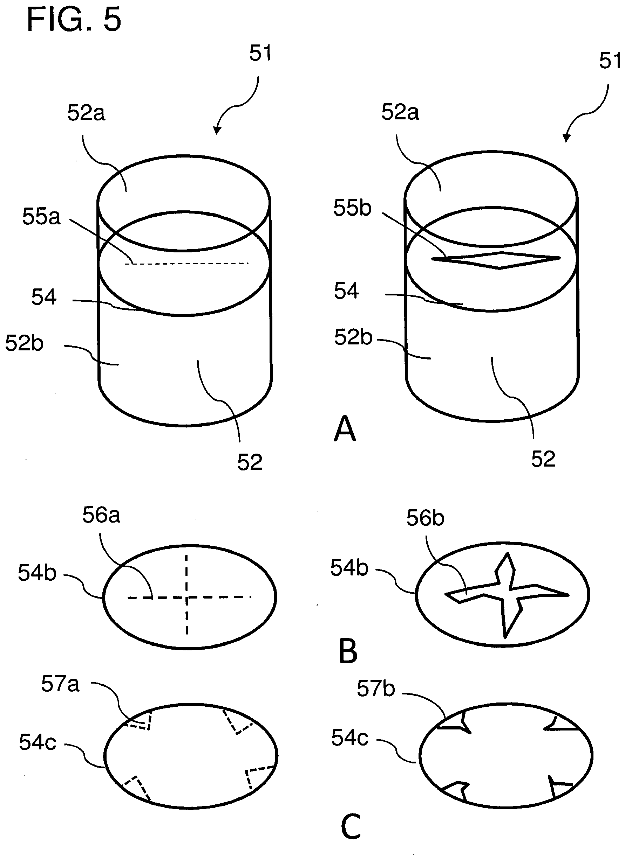

Figur 5 illustriert schematisch verschiedene Ausführungsvarianten einer Kammer bzw. eines Behälters, die als Indikatoreinrichtung zur Temperaturüberwachung ausgelegt sind.

Figur 5A zeigt einen Behälter 51, beispielsweise einen Zylinder, der in seinem Innenraum 52 zwei Teilvolumina 52a und 52b ausbildet, die durch eine Barriere 54 getrennt sind. In dem oberen Teilvolumen 52a wird eine Indikatorsubstanz (nicht dargestellt) eingebracht. Die Barriere 54 kann eine Membran oder Folie sein, die Sollbruchstellen, schematisch dargestellt durch die gestrichelte Linie 55a, aufweist, an denen diese Barriere 54 bei Abkühlung auf die Lagertemperatur zerreißt. Die Barriere ist so ausgelegt, dass sie erst bei einer Temperatur unterhalb der Schmelztemperatur der Indikatorsubstanz, die sich im Teilvolumen 52a befindet, zerreißt. Auf diese Weise kann zunächst nur das obere Teilvolumen 52 mit flüssiger Indikatorsubstanz befüllt und anschließend abgekühlt werden.

Die Barriere 54 kann auch aus einem Material bestehen, das stärker bei Abkühlung schrumpft als das Zylindermaterial und daher mehr oder minder irregulär reißt.

Der mit Indikatorsubstanz befüllte Behälter 51 kann dann an einem Probenbehälter, in dem eine Kryoprobe gelagert ist, angeordnet werden und zusammen mit diesem auf Lagertemperatur, die unterhalb der Schmelztemperatur der Indikatorsubstanz liegt, abgekühlt werden.

Bei der Abkühlung reißt die Barriere, so dass die im Teilvolumen 52a befindliche Indikatorsubstanz im flüssigen Aggregatszustand über die durch die mindestens eine Rissstelle gebildete Öffnung 55b langsam von dem ersten Teilbereich 52a in den zweiten Teilbereich 52b gelangen kann.

Figur 5B bzw. Figur 5C zeigen weitere Ausführungsformen einer Barriere 54b bzw. 54c mit einer derartigen Sollbruchstelle 56a bzw. 57a. Links in den Figuren 5B und 5C sind jeweils die Sollbruchstellen bei intakter Barriere vor dem Abkühlen dargestellt, rechts aufgerissen nach dem Abkühlen (Öffnungen 56b bzw. 57b).

Solange die Testflüssigkeit im oberen Teilvolumen 52a gefroren bleibt, erfolgt kein Durchtritt durch die Öffnung 55b, 56b oder 57b in das Volumen 52b. Bei korrekter Lagerung erscheint die Füllung getrennt wie vor dem Einfrieren und kann bei der Lagertemperatur beliebig oft kontrolliert werden. Wird eine solche Probe aufgetaut, wird die Phase im oberen Teilvolumen 52a flüssig und dringt über die Öffnung 55b, 56b oder 57b in das Volumen 52b ein. Auf diese Weise kann getestet werden, ob die Barriere 54 bei der Lagertemperatur ordnungsgemäß gerissen war, das System also in der gewünschten Weise funktioniert hat.

Über die in den Figuren 1 bis 5 gezeigten Varianten sind nun auch komplexere Systeme mit verschiedenen solchen Elementen und Temperaturerfassungen oder Mischungen von zwei oder mehreren Flüssigkeiten bei Erwärmung aufbaubar.

Figur 6 illustriert anhand eines Ablaufdiagramms ein Verfahren zur Temperaturüberwachung einer kryokonservierten biologischen Probe. In Schritt S1 wird eine Vorrichtung zur Temperaturüberwachung bereitgestellt, beispielsweise eine der Vorrichtungen 10, 20, 30, oder 40. Hierbei ist je nach Temperaturgrenzwert, der bei der Kryolagerung überwacht werden soll, eine geeignete Flüssigkeit oder ein Flüssigkeitsgemisch als Indikatorsubstanz 7 auszuwählen.

Über die Auswahl geeigneter Flüssigkeiten und das Mischungsverhältnis von Flüssigkeiten kann deren Schmelzpunkt auf einen gewünschten Wert in einem Bereich von -20 °C bis -140 °C festgelegt werden.

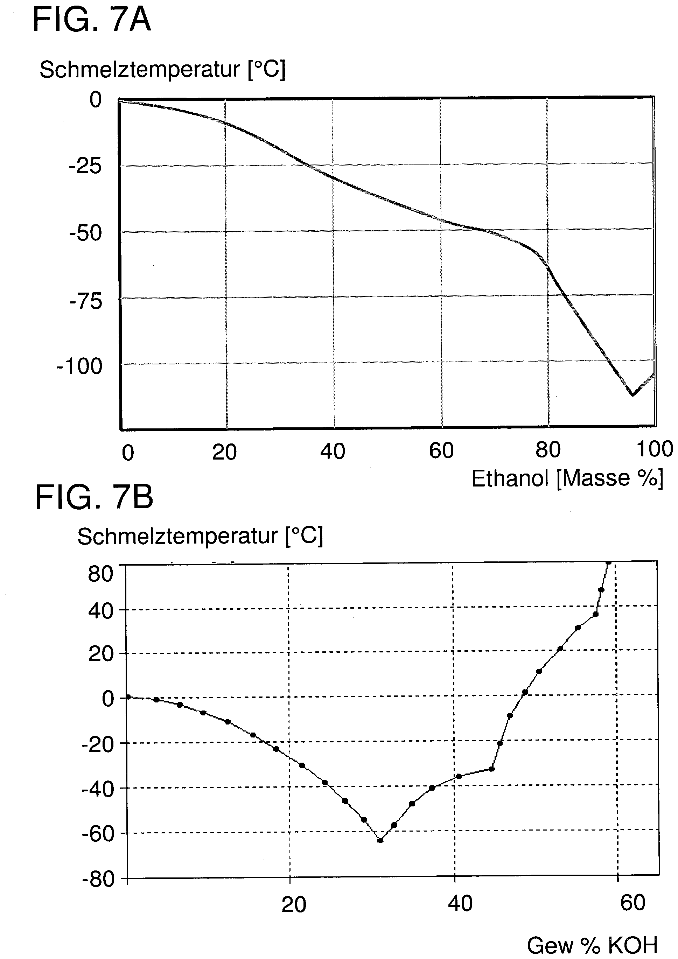

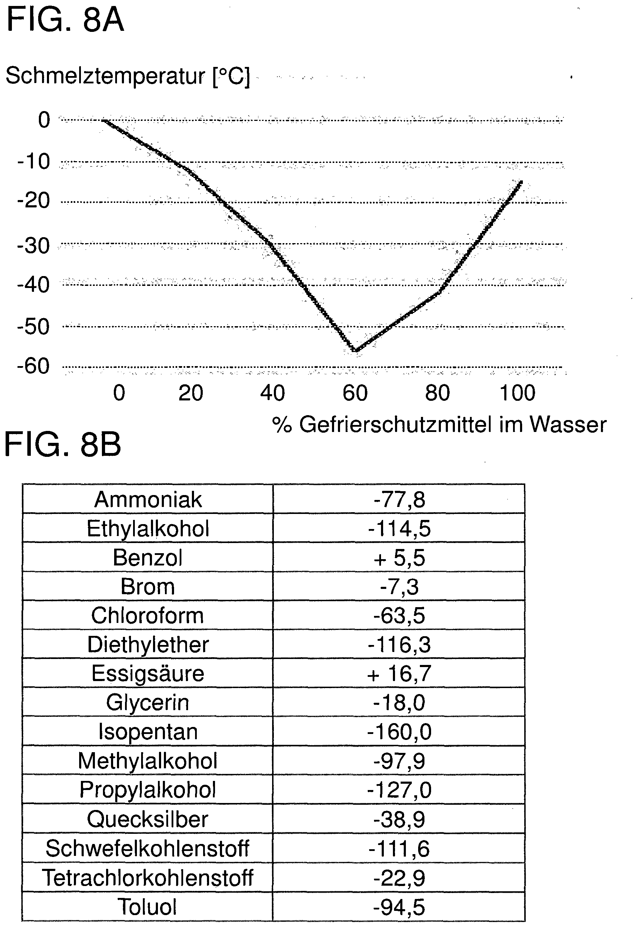

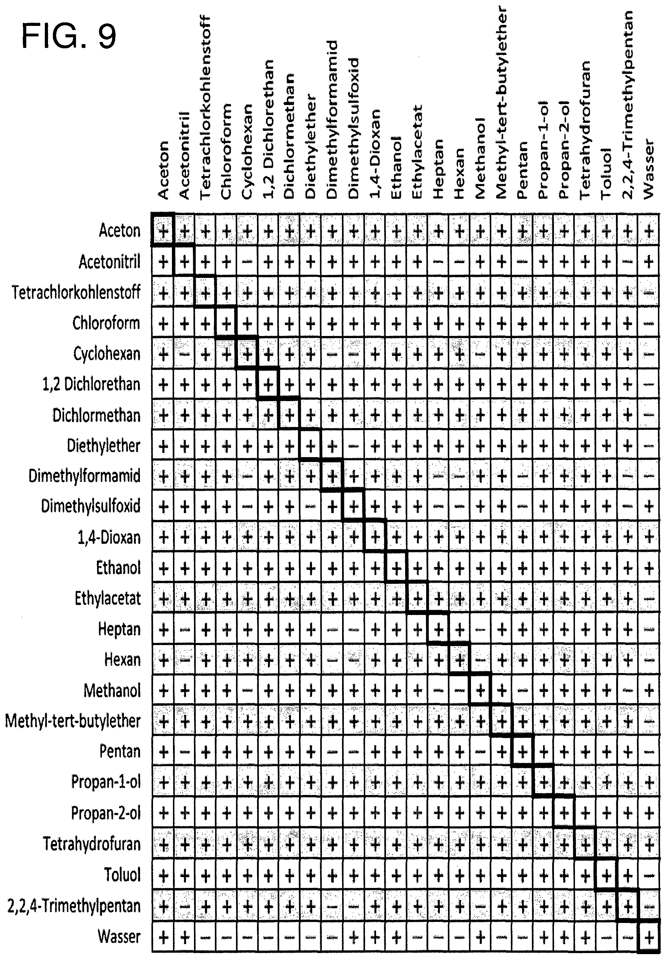

Beispielhaft ist in Figur 7A der Verlauf des Schmelzpunktes als Funktion des Mischungsverhältnisses aus einem Alkohol und Wasser angegeben, mit dem bei moderater Viskositätserhöhung mit abfallender Temperatur ein Temperaturbereich zwischen 0 °C und -118 °C abgedeckt werden kann. Soll z. B. ein Temperaturgrenzwert von -118 °C überwacht werden, kann der Ethanol-Anteil auf 93,5 % festgelegt werden. Schmelzpunkte bis zu einem Wert von knapp unter -60 °C können auch durch Zumischung von Kaliumhydroxid (KOH) zu Wasser eingestellt werden, was in Figur 7B anhand eines Schmelzdiagramms gezeigt ist. Auch eine Mischung aus Waser und Gefrierschutzmittel kann als Indikatorsubstanz verwendet werden, was durch das Schmelzdiagramm der Figur 8A illustriert ist. Die Tabelle der Figur 8B führt Gefrierpunkte/Schmelzpunkte weiterer reiner Flüssigkeiten auf, die alleine oder als Mischung mit einer anderen Flüssigkeit als Indikatorsubstanz verwendet werden können. Weitere als Indikatorsubstanz geeignete Flüssigkeitsgemische sind Chloroform-Zyklohexan-Gemische oder andere mischbare Flüssigkeiten, die z. B. aus der Mischbarkeitsmatrix von Lösemitteln der Figur 9 entnommen werden können.

Vornehmlich werden Flüssigkeiten und Plastikmaterialien mit guter Benetzbarkeit und niedriger Viskosität bei niedrigen Temperaturen ausgewählt, um die Lageveränderung möglichst umfangreich und die Zusatzkompartimente klein zu gestalten.

Falls mehrere Temperaturgrenzwerte bei der Kryolagerung überwacht werden sollen bzw. falls die erreichten Temperaturintervalle, in die die Probe gelangt ist, genauer eingegrenzt werden sollen, können entsprechend mehrere verschiedene Indikatorsubstanzen mit verschiedenen Schmelzpunkten verwendet werden, die dann in verschiedenen Kammern im oder am Probenbehälter angebracht werden.

Die Indikatorsubstanz kann je nach Ausführungsform der Vorrichtung zusammen oder getrennt mit der Vorrichtung abgekühlt und in einen gefrorenen Zustand gebracht werden, wie dies beispielhaft vorstehend im Rahmen der Figuren beschrieben wurde.

In Schritt S2 wird die Vorrichtung mit einer Kryoprobe im Aufnahmeraum des Probenbehälters bei einer Lagertemperatur unterhalb der Schmelztemperatur gelagert.

Nachfolgend kann mittels der Indikatorsubstanz zu einem beliebigen Zeitpunkt während des Lagervorgangs überprüft werden, ob eine unerwünschte, wenn auch nur zeitweise Erwärmung der Kryoprobe stattgefunden hat (Schritt S3). Hierzu wird geprüft, ob sich zu einem späteren Zeitpunkt im zweiten Teilbereich der Kammer Indikatorsubstanz befindet. Ist dies der Fall, kann auf ein Überschreiten der Schmelztemperatur der Indikatorsubstanz und somit der zu überwachenden Grenztemperatur geschlossenen werden, insbesondere auch dann, wenn die Überschreitung nur kurzzeitig aufgetreten ist.

Nachfolgend wird noch ein Kontrollablauf beschrieben, der sich für eine Vorrichtung zur Temperaturüberwachung eignet, die ein Trennelement als Barriere oder eine Abdeckung als Teil der Barriere in der Kammer aufweist, die aufgrund der thermischen Schrumpfung reißt und damit die Öffnungen für das Eindringen der Indikatorflüssigkeit in den zweiten Teilbereich der Kammer bildet.

Der nachfolgend beschriebene Kontrollablauf gestattet es bei jeder Probe, die Funktionstüchtigkeit der Vorrichtung, insbesondere der als Indikatoreinrichtung dienenden Kammer, zu kontrollieren.

In einem ersten Prüfschritt kann die Indikatoreinrichtung in Form der Kammer mit der Barriere bei Raumtemperatur betrachtet werden, bevor die Probe eingefroren wird. Die Indikatorsubstanz befindet sich dann im flüssigen Aggregatszustand auf der einen Seite der Barriere, d. h. im ersten Teilbereich der Kammer, die Barriere ist intakt, und es befindet sich keine Indikatorsubstanz im darunter befindlichen zweiten Teilbereich. Ist das nicht der Fall, kann diese Kammer nicht benutzt werden, da sie bereits defekt ist. Ist die Kammer beispielsweise in den Deckel integriert, kann dieser nicht mehr benutzt werden und wird durch einen anderen ausgetauscht.

Nach dem Abkühlen unterhalb die Schmelztemperatur der Indikatorsubstanz sollte die Barriere durch die thermische Kontraktion geöffnet sein, z. B. an vorgeschwächten Stellen, die Indikatorflüssigkeit ist jedoch gefroren, so dass das Bild dem vor dem Einfrieren entsprechen muss. Dies kann in einem zweiten Prüfschritt geprüft werden. Ist das nicht der Fall, ist eine neue Kammer, z. B. ein neuer Deckel, zu benutzen.

Im tiefgefrorenen Zustand kann nun zu beliebigen Zeitpunkten kontrolliert werden, ob eine Erhöhung der Temperatur über den Schmelzpunkt der Indikatorsubstanz erfolgt ist. Dann sollte diese durch die Barriere in den zweiten Teilbereich eingetreten sein. Dies ist in einem dritten Prüfschritt leicht erkennbar, insbesondere wenn die Indikatorsubstanz einen Farbstoff als Indikatorzusatz enthält.

Entnimmt man eine tiefgefrorene Probe zur Nutzung, die keine Überschreitung der Schmelztemperatur der Indikatorsubstanz erlebt hat, dann sieht der Zustand der Kammer so aus, wie unter den ersten beiden Prüfschritten beschrieben, d. h., es befindet sich keine Indikatorsubstanz im zweiten Teilbereich der Kammer. Es könnte allerdings sein, dass das Trennelement gar nicht gerissen ist und die Barriere somit nicht durchlässig geworden ist und damit die Überschreitung der Indikatorsubstanzschmelztemperatur nicht angezeigt wurde. Dies lässt sich prüfen, indem dass die Kammer nach dem Auftauen eine gewisse Zeit liegengelassen wird. Beispielsweise kann bei einer in den Deckel integrierten Kammer nur der Deckel liegengelassen werden, während die Probe weiter verarbeitet werden kann. War das Trennelement ordnungsgemäß gerissen und die Temperatur lag die ganze Zeit unterhalb der Schmelztemperatur, dann wird nun bei Raumtemperatur in jedem Fall die Indikatorflüssigkeit durch die Barriere in das Testvolumen, d. h. in den zweiten Teilbereich, diffundieren. Auf diesem Wege ist die Funktionsfähigkeit der Indikatoreinrichtung bewiesen und eindeutig belegt, dass die Lagertemperatur über die gesamte Zeit den Vorgaben entsprochen hat.

Der vorstehende Kontrollablauf funktioniert analog für den Fall, dass die Barriere durch eine Abdeckung gebildet ist, die auf dem Material mit flüssigkeitsaufnehmender Struktur angeordnet ist und ausgelegt ist, bei Abkühlung der Vorrichtung auf eine Lagertemperatur, die unterhalb der Schmelztemperatur der Indikatorsubstanz liegt, von einem ersten Zustand der Abdeckung, in dem diese gegenüber der Indikatorsubstanz impermeabel ist, irreversibel in einen zweiten Zustand der Abdeckung, in dem diese für die Indikatorsubstanz permeabel ist, überzugehen.

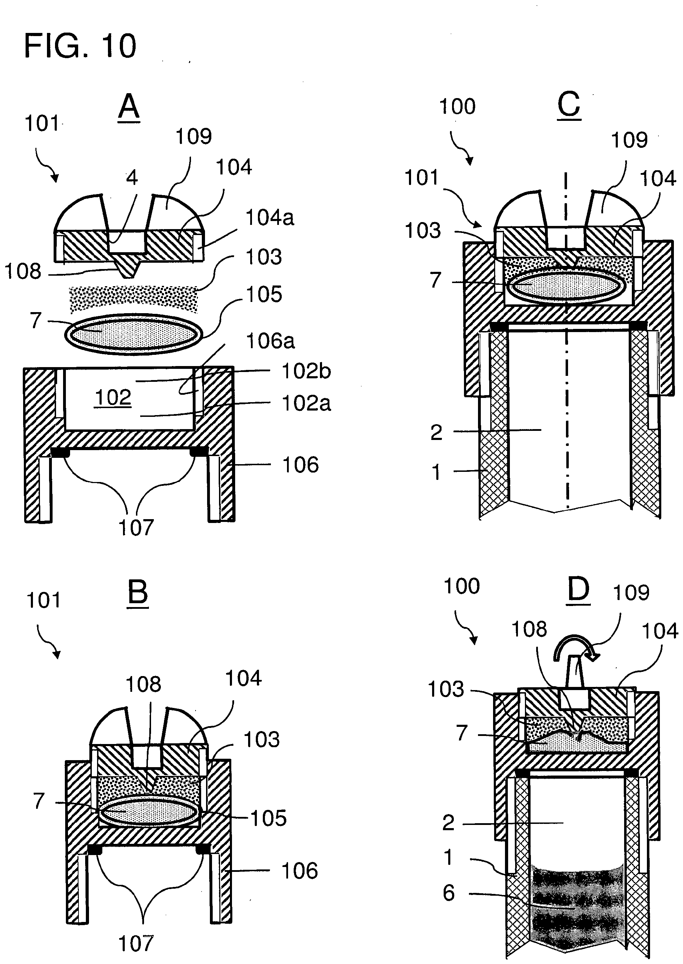

Figur 10 illustriert in mehreren Querschnittsansichten ein weiteres Ausführungsbeispiel 100 einer Vorrichtung zur Temperaturüberwachung. Die Ansicht A zeigt dabei eine Explosionsdarstellung eines Deckels 101 eines Kryoröhrchens 1, in dem eine Einrichtung zur Temperaturüberwachung integriert ist. Die Vorrichtung 100 umfasst das Kryoröhrchen 1 mit einem Aufnahmeraum 2 zur Aufnahme einer biologischen Probe 6 sowie den Deckel 101, der eine Kammer 102 aufweist, die eine Indikatorsubstanz 7 enthält, die durch eine Barriere im nicht scharfgeschalteten Zustand von einem Saugmaterial 103 getrennt ist. Der Deckel 101 wird nachfolgend auch als Temperatur-sensitiver (T-sensitiver) Deckel bezeichnet. Die Ansicht B zeigt den Deckel 101 im montierten Zustand, die Ansicht C illustriert ein verkaufsfertiges Kryoröhrchen 1 mit einem Aufnahmeraum 2 zur Aufnahme einer biologischen Probe 6 und mit dem aufgeschraubten T-sensitiven Deckel 101. Die Ansichten A bis C zeigen die Vorrichtung 100 im noch nicht scharfgeschalteten Zustand.

Der T-sensitive Deckel 101 umfasst vier Teile: einen Schraubeinsatz 104, nachfolgend als Einschraubteil 104 bezeichnet, einen Einsatz mit flüssigkeitsaufnehmender Struktur 103, nachfolgend als Saugmaterial 103 bezeichnet, der in der Lage sein muss, flüssige Indikatorsubstanz 7 aufzusaugen, einen Behälter 105 für die Indikatorsubstanz 7, hier beispielhaft ausgeführt als Plastikkissen 105, der gefärbte Flüssigkeit als Indikatorsubstanz 7 enthält, wenn diese nicht gefroren ist, und einen Grundkörper 106 zum Aufschrauben auf ein handelsübliches Kryoröhrchen 1, hier ausgeführt als Plastikkappe 106.

Das Plastikkissen 105 bildet im nicht scharfgeschalteten Zustand der Vorrichtung 100 eine dichte Hülle für die darin befindliche Indikatorsubstanz 7 aus und damit eine Barriere, die im nicht scharfgeschalteten Zustand verhindert, dass die Indikatorsubstanz 7 in Kontakt mit dem Saugmaterial 103 kommen kann. Die Ausführung als Plastikkissen bietet den Vorteil, dass dieses sehr kostengünstig herstellbar ist.

Das Einschraubteil 104 ist an zumindest einer Stelle transparent oder semi-transparent ausgeführt, so dass von oben durch das Einschraubteil 104 zumindest ein Teil des Saugmaterials 103 sichtbar ist. Das Einschraubteil 104 kann beispielsweise hierzu aus einem transparenten oder semitransparenten Material hergestellt sein. Ferner kann der Grundkörper 106 hierzu aus einem transparenten oder semitransparenten Material hergestellt sein. Auf diese Weise kann durch einfache Sichtprüfung von oben kontrolliert werden, ob sich der Farbzustand des Saugmaterials 103 geändert hat. Dies ist z. B. der Fall, wenn die gefärbte Indikatorsubstanz 7 in das Saugmaterial 103 einpenetriert ist und diese dadurch verfärbt, was nachfolgend noch beschrieben wird.

Der Grundkörper bzw. die Plastikkappe 106 hat einen H-förmigen Querschnitt, wodurch zwei zylinderförmige Hohlräume ausgebildet werden. Der obere Hohlraum 102 bildet eine Kammer mit einem ersten Teilbereich 102a, in dem sich der Behälter 105 mit Indikatorsubstanz 7 befindet, und mit einem zweiten Teilbereich 102b, in dem sich das Saugmaterial 13 befindet.

Der untere Hohlraum dient zur Aufnahme eines oberen Endabschnitts des Kryoröhrchens 1, um dieses dicht zu verschließen. Abgedichtet wird das Kryoröhrchen 1 mit einem Dichtungsring 107. Im Einschraubteil 103 befindet sich ein Eingriff 4, z. B. ein 6-Kantloch, über das das Einschraubteil 104 in den Grundkörper 106 eingedreht werden kann. Das Einschraubteil 104 kann auch Flügelblätter 109 aufweisen, über die das Einschraubteil 104 ebenfalls eingedreht werden kann. Zum Einschrauben ist am Einschraubteil 104 ein Außengewinde 104a vorgesehen, das in ein korrespondierendes Innengewinde 106a des Grundkörpers 106 eingreift, das an einer seitlichen Wandung des oberen Hohlraums 102 vorgesehen ist.

Wie bereits erwähnt zeigen die Ansichten A bis C die Vorrichtung 100 im noch nicht scharfgeschalteten Zustand. Dies bedeutet, dass das mit gefärbter Indikatorsubstanz gefüllte Plastikkissen 105 unzerstört und dicht ist, so dass Indikatorsubstanz 7 im flüssigen Zustand nicht austreten kann. Im Innern des Plastikkissens 105 befindet sich die Indikatorsubstanz 7 zunächst in flüssiger Form. Das darüber befindliche Saugmaterial 103 steht aufgrund des intakten Plastikkissens 105 nicht in Kontakt mit der Indikatorsubstanz 7. Die Indikatorsubstanz kann beispielsweise eine der vorstehend genannten Substanzen enthalten und durch ein Gemisch dieser Substanzen gebildet sein. Beispielhaft kann die Indikatorsubstanz 7 ferner einen Farbstoff als Indikatorzusatz enthalten, z. B. den Farbstoff Rhodamin B, sodass diese rot gefärbt ist.

Das Einschraubteil 104 ist zunächst halb eingeschraubt (nicht scharfgeschalteter Zustand). Um sie weiter einzudrehen, z. B. eine viertel oder halbe Drehung, muss eine Plastiksperre im Gewinde 103a, 106a durchbrochen werden. Damit ist sichergestellt, dass die Einrichtung zur Temperaturüberwachung und/oder der T-sensitive Deckel nicht vor der Nutzung aktiviert werden. Sollte dies dennoch vor der Kryolagerung der Vorrichtung 100 bzw. der Probe 6 passieren, wird das in dem Grundkörper 106 befindliche Saugmaterial 103 rot und darf folglich nicht eingesetzt werden. Der Transport und die Zwischenlagerung des Deckels 101 im nicht scharfgeschalteten Zustand können über beliebige Zeit erfolgen, was den Handel und die Vorratshaltung befördert.

Die Ansicht D der Figur 10 zeigt die Aktivierung (Scharfschaltung) der Vorrichtung nach Erreichen der Lagertemperatur, vorliegend z. B. < -140°C.

Die Indikatorsubstanz 7 ist so gewählt, dass sie bei der Lagertemperatur in dem Behälter 105 nicht mehr flüssig, sondern bereits erstarrt ist. Die Aktivierung (Scharfschaltung) der Vorrichtung erfolgt durch Einschrauben des Einschraubteils 104 bei gefrorener Indikatorsubstanz 7. Das Einschraubteil 104 weist an seiner unteren, dem Saugmaterial 103 zugewandten Seite einen abragenden Vorsprung 108 auf, z. B. in Form einer Spitze oder eines Dorns. Durch das Einschrauben des Einschraubteils 104 wird der Vorsprung 108 in das Plastikkissen 105 gebohrt und zerstört dieses. Dadurch kommt das Saugmaterial 103 in direkten Kontakt mit der roten Indikatorsubstanz 7. Diese ist bei der Lagertemperatur so zäh oder auch fest, dass sie nicht in das Saugmaterial 103 gezogen wird. Auch die noch immer ablaufenden thermischen Stöße reichen nicht für eine Diffusion aus.

Die Vorrichtung zur Temperaturüberwachung ist nun aktiviert (scharf). Sobald eine Übergangstemperatur während der Kryolagerung, die den Schmelzpunkt des Gemisches der Indikatorsubstanz 7 angibt, überschritten wird, wird die gefrorene Indikatorsubstanz 7 flüssig. Mit steigender Temperatur nimmt die Viskosität ab, bis bei der Schwellentemperatur eine Schwelle überschritten wird, ab der das Saugmaterial 103 über Kapillarkräfte die Flüssigkeit einzieht und damit deren Farbe annimmt. Dieser Prozess ist irreversibel, d. h., selbst nach einem nachfolgenden erneuten Gefrieren der Indikatorsubstanz 7 bleibt die rote Verfärbung des Saugmaterials 103 erhalten. Wird somit nachträglich bei einer Sichtkontrolle festgestellt, dass das Saugmaterial 103 eine rote Verfärbung aufweist, kann gefolgert werden, dass die Schmelztemperatur der Indikatorsubstanz und ferner eine etwas höhere Temperatur als die der Schmelztemperatur ebenfalls überschritten wurden, bei der die Viskosität der Indikatorsubstanz 7 so niedrig geworden ist, dass sie über Kapillarkräfte in das Saugmaterial 103 gezogen wurde. Die Art und Dicke des Saugmaterials 103 bestimmt, wie schnell die erkennbare Färbung auftritt. Das Saugmaterial 103 kann beispielsweise Filterpapier, z. B. wie das einer herkömmlichen Küchenrolle oder eines Zigarettenfilterpapiers, ein Presskörper, Zellulosescheiben, z. B. Taschentuchzellulosescheiben, Gips oder Kreidestaub sein.

Was für das Saugmaterial 103 und die Indikatorsubstanz 7 im Innern des Grundkörpers 106 gilt, trifft auch für die Temperatur in der Bioprobe 6 zu. Ein rot verfärbtes Saugmaterial zeigt somit an, dass auch die Bioprobe 6 die vorgenannten Temperaturen zumindest temporär überschritten hat. Da dieser Prozess auch bei erneutem Tiefkühlen nicht wieder aufgehoben werden kann, bewahrt die Vorrichtung 1 somit die Information der unerlaubten Erwärmung.

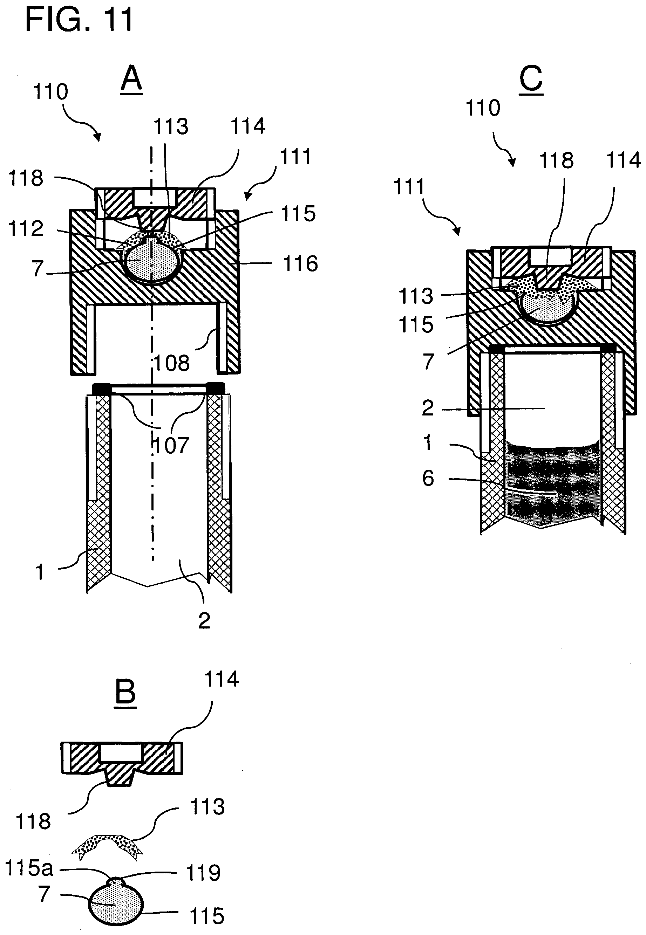

Figur 11 illustriert in mehreren Querschnittsansichten ein weiteres Ausführungsbeispiel 110 einer Vorrichtung zur Temperaturüberwachung. Die Vorrichtung 110 umfasst wiederum ein Kryoröhrchen 1 mit einem Aufnahmeraum 2 zur Aufnahme einer biologischen Probe 6 sowie eine Deckel 111. Der T-sensitive Deckel umfasst wiederum vier Teile: einen Schraubeinsatz 114, nachfolgend als Einschraubteil 114 bezeichnet, einen Einsatz mit flüssigkeitsaufnehmender Struktur 113, nachfolgend als Saugmaterial 113 bezeichnet, der in der Lage sein muss, flüssige Indikatorsubstanz 7 aufzusaugen, und einen Behälter 105 für die Indikatorsubstanz 7 und einen Grundkörper 116 zum Aufschrauben auf ein handelsübliches Kryoröhrchen 1, hier ausgeführt als Plastikkappe 116.

Die Ansicht A zeigt hierbei den Deckel 111 im nicht scharfgeschalteten Zustand vor der Montage auf dem Kryoröhrchen 1. Die Ansicht B zeigt eine Explosionsdarstellung von einzelnen Komponenten des Deckels 111. Die Ansicht C zeigt die Vorrichtung 110 im scharfgeschalteten Zustand.

Im Unterschied zu der in Figur 10 dargestellten Ausführungsvariante ist die Barriere nun nicht durch ein Plastikkissen gebildet, sondern durch eine Glaskugel 115, die Indikatorsubstanz 7 enthält. Zum Befüllen der Glaskugel 115 mit Indikatorsubstanz 7 kann diese eine Öffnung 115a aufweisen, die nach Befüllen mit der Indikatorsubstanz 7 durch einen Verschluss 119 verschlossen wird, beispielsweise durch Verkleben der Öffnung 115a mit einem 2-Komponentenkleber oder durch Zuschweißen unter Kühlung der Indikatorflüssigkeit 7. Auf die so verschlossene Glaskugel 115 wird dann wiederum ein Saugmaterial 113 gelegt, die in diesem Zustand (nicht scharfgeschalteter Zustand) nicht im Kontakt mit der Indikatorsubstanz 7 ist.

Zum Aktivieren bzw. zum Scharfschalten der Vorrichtung 110 wird diese zunächst auf Lagertemperatur der Kryolagerung abgekühlt, bei der die Indikatorsubstanz 7 gefroren ist. Anschließend wird, analog wie zuvor für die Ausführungsvariante der Figur 10 beschrieben, das Einschraubteil 114 so weit in die Plastikkappe 116 eingedreht, bis die die Indikatorsubstanz 7 von dem Saugmaterial 113 trennende Barriere zerstört wird. Bei dem in Figur 11 gezeigten Ausführungsbeispiel geschieht dies dadurch, dass durch ein Drehen des Einschraubteils 114 ein in Richtung der Glaskugel 115 abragender Vorsprung 118 des Einschraubteils die Glaskugel 115 zerdrückt. Dieser aktivierte bzw. scharfgeschaltete Zustand der Vorrichtung 110 ist in Abbildung C dargestellt. Die Indikatorsubstanz 7 ist jedoch so gewählt, dass diese bei der Lagertemperatur so zäh oder auch fest ist, dass sie nicht in das Saugmaterial 113 gezogen wird. Auch die noch immer ablaufenden thermischen Stöße reichen nicht für eine Diffusion aus. Erst wenn nachfolgend eine Übergangstemperatur während der Kryolagerung, die z. B. den Schmelzpunkt des Gemisches der Indikatorsubstanz 7 angibt, überschritten wird, wird die gefrorene Indikatorsubstanz 7 flüssig. Mit steigender Temperatur nimmt die Viskosität zunehmend ab, bis bei der Schwellentemperatur eine Schwelle überschritten wird, ab der das Saugmaterial 113 über Kapillarkräfte die flüssige Indikatorsubstanz 7 zumindest teilweise einzieht und damit deren Farbe annimmt.

Ein besonderer Vorteil der Ausführung der Barriere als Glaskugel 115 ist, dass diese beim Zerdrücken ein Knackgeräusch macht, so das beim Aktivieren der Vorrichtung 110 gleichzeitig ein akustisches Feedbacksignal erzeugt wird, das die Vorrichtung nun scharf geschaltet ist.

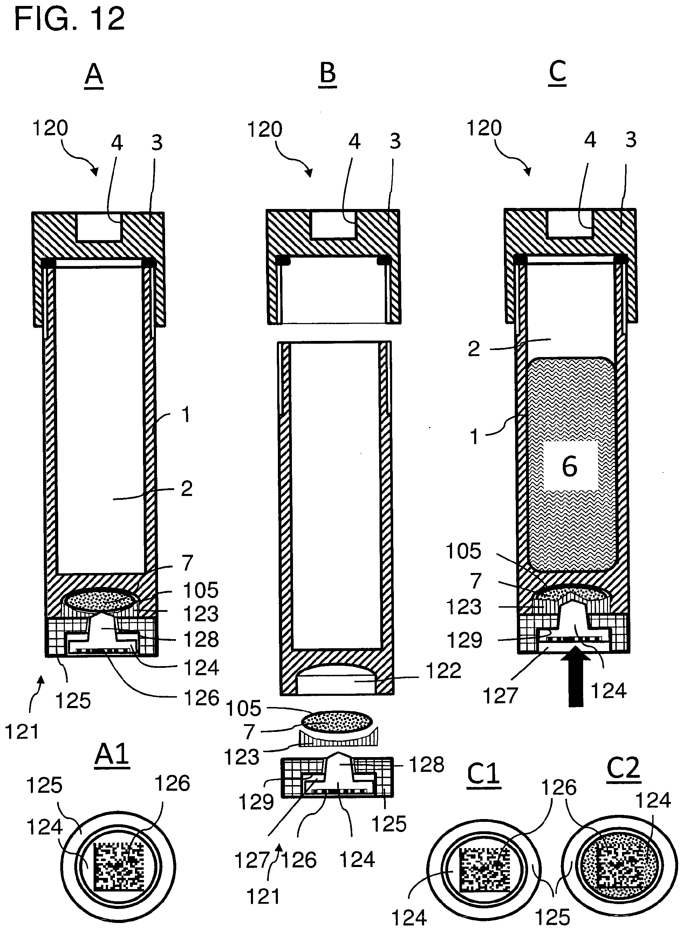

Figur 12 illustriert in mehreren Ansichten ein weiteres Ausführungsbeispiel 120 einer Vorrichtung zur Temperaturüberwachung. Die Ansicht A zeigt hierbei eine Querschnittsansicht der Vorrichtung 120 bzw. des kompletten Kryröhrchens vor der Aktivierung (im nicht scharfgeschalteten Zustand). Die Ansicht A1 zeigt eine Unteransicht des Kryoröhrchens 120. Die Ansicht B zeigt eine Querschnittsansicht der Vorrichtung 120 in einer Explosionsdarstellung zur Illustration der Einzelteile der Vorrichtung 120. Die Ansicht C zeigt einen Querschnitt der Vorrichtung 120 im aktivierten Zustand (scharfgeschalteter Zustand).

Die Vorrichtung 120 umfasst wiederum ein Kryoröhrchen 1 mit einem Aufnahmeraum 2, in den die biologische Probe 6 gefüllt wird, sowie einen aufschraubbaren Deckel 103, der hier in üblicher Weise ausgeführt ist und einen Eingriff 4 aufweisen kann. Die Vorrichtung umfasst wiederum eine Einrichtung zur Temperaturüberwachung, ähnlich zu derjenigen, die in Figur 10 oder Figur 11 zur Verwendung im Schraubdeckel beschrieben wurde. Die Besonderheit dieses Ausführungsbeispiels liegt darin, dass die Einrichtung zur Temperaturüberwachung in einen unteren Teil 121 des Kryoröhrchens 1 integriert ist, d. h. am zum Deckel gegenüberliegenden Endbereich des Kryoröhrchens 1. Der Vorteil dieser Ausführung besteht darin, dass sich im Unterschied zur Schraubdeckelvariante keine einfache Austauschmöglichkeit der Einrichtung zur Temperaturüberwachung mehr ergibt, da die biologische Probe 6 sich in diesem unteren Teil festgefroren befindet und mit entfernt würde.

Die in den unteren Teil des Kryoröhrchens integrierte Einrichtung zur Temperaturüberwachung umfasst ein Saugmaterial 123, eine Indikatorsubstanz 7, die zunächst im flüssigen Zustand in einem abgeschlossenen ellipsoiden Behälter 105, z. B. aus Plastik oder Glas, aufbewahrt ist, und ein Plastikbodenteil 125 am Boden des Kryoröhrchens 1, der eine Öffnung 127 aufweist, in der sich ein einmal nach oben eindrückbares rundes Teil 124 befindet, an dessen Unterseite ein konventioneller 2D-Barcode 126 aufgeprägt ist. Auf der zum Barcode abgewandten Seite weist das Teil 124 einen zylindrischen, leicht kegelförmigen Dorn 128 auf. Das Teil 124 wird nachfolgend auch als Stößel 124 bezeichnet. Der Stößel 124 und vorzugsweise auch das Plastikbodenteil 125 sind aus einem transparenten oder semitransparenten Material hergestellt. Die Indikatorsubstanz 7 kann wieder einen Farbstoff als Indikatorzusatz enthalten, z. B. den Farbstoff Rhodamin B, so dass diese rot gefärbt ist.

Im Fertigungsprozess werden die Teile wie folgt montiert: Der Behälter 105 mit Indikatorsubstanz 7 wird in eine Vertiefung 122 am Boden des Röhrchens 1 eingefügt. Die Vertiefung ist an ihrer in Figur 12 oben liegenden Seite formkorrespondierend zum Indikatorsubstanzbehälter 105 gekrümmt. Darüber wird das Saugmaterial 123 gelegt. Die Vertiefung 122 bildet somit eine Kammer zur Aufnahme des Indikatorsubstanzbehälters 105 und des Saugmaterials 123. Nun wird das Plastikbodenteil 125 aufgesetzt und fest und vorzugsweise unlösbar mit dem Kryoröhrchen 1 verbunden, z. B. durch Anschweißen oder Ankleben.

Der Stößel 124 mit dem Barcode 126 wird so weit eingeschoben, dass er gerade mit dem Dorn 128 in das Saugmaterial 123, nicht aber in den Indikatorsubstanzbehälter 105 eindringt. Dies kann z. B. dadurch erreicht werden, dass das Kryoröhrchen 1 senkrecht auf den Stößel 124 aufgedrückt wird, bis er plan mit der Bodenfläche des Plastikbodenteils 125 abschließt, wie es in der Ansicht A gezeigt ist. Der Nutzer erhält, wie gegenwärtig auch, zwei Teile montiert: den Deckel 3 und das gefügte Unterteil 1, 121 bestehend aus dem Kryoröhrchen, dem Stößel 124, dem Indikatorsubstanzbehälter 105, dem Saugmaterial 123, dem Plastikbodenteil 125 und dem Barcode 126. In diesem Zustand ist die Einrichtung zur Temperaturüberwachung am Boden nicht aktiviert (nicht scharfgeschaltet).

Bei Nutzung füllt der Anwender die Bioprobe 6 in das Unterteil 1, 121, kühlt das Röhrchen auf die Lagertemperatur und drückt dann am Boden den Stößel 124 mit Kraft bis zum Anschlag 129 nach oben (illustriert durch den Pfeil in Ansicht C).

Dadurch wird das Behältnis 105 der gefrorenen Indikatorflüssigkeit 7 zerstört und das Saugmaterial 123 auf diese gedrückt. Der etwas konische Stößel 124 dichtet die Öffnung 127, durch die er sich bewegt, ab. Optional kann auch noch eine Beschichtung auf dem Stößel 124 angebracht werden, die zu einem gasdichten Abschließen führt. Nun ist die Vorrichtung 120 aktiviert.

Die Indikatorsubstanz 7 ist wiederum so gewählt, dass sie bei der Lagertemperatur in dem Behälter 105 nicht mehr flüssig, sondern bereits erstarrt ist bzw. zumindest so zäh oder auch fest, dass sie nicht in das Saugmaterial 123 gezogen wird. Auch die noch immer ablaufenden thermischen Stöße reichen nicht für eine Diffusion aus.

Sobald eine Übergangstemperatur während der Kryolagerung, die den Schmelzpunkt der Indikatorsubstanz 7 angibt, überschritten wird, wird die gefrorene Indikatorsubstanz 7 flüssig. Mit steigender Temperatur nimmt die Viskosität ab, bis eine Schwellentemperatur überschritten wird, ab der das Saugmaterial 123 über Kapillarkräfte die Indikatorflüssigkeit 7 einzieht und damit deren Farbe annimmt. Dieser Prozess ist irreversibel, d. h., selbst nach einem nachfolgenden erneuten Gefrieren der Indikatorsubstanz 7 bleibt die rote Verfärbung des Saugmaterials 123 erhalten.

Wird die zu überwachende erste oder zweite Schwellwerttemperatur nicht überschritten, bleibt der Boden des Kryoröhrchens in der Farbe unverändert (z. B. weiß, gezeigt in Ansicht C1). Dies ist in Ansicht C1 illustriert, die eine Unteransicht des Kryoröhrchens 120 in einem Zustand zeigt, in dem die Indikatorsubstanz nicht in das Saugmaterial gezogen wurde. Wird die zweite Schwellwerttemperatur bei der Lagerung der aktivierten Vorrichtung 120 jedoch überschritten, saugt sich das Saugmaterial 123 voll, und der Boden 121 des Kryoröhrchens, insbesondere der Stößel 124, erscheint gefärbt, z. B. rot gefärbt. Dies ist in Ansicht C2 illustriert, die eine Unteransicht des Kryoröhrchens 120 in einem Zustand zeigt, bei dem die Indikatorsubstanz 7 in das Saugmaterial gezogen wurde.

Diese Ausführung ist auch deshalb vorteilhaft, weil derartige Barcode-Röhrchen bereits weit verbreitet sind und daher die Färbung am Boden leicht ausgelesen werden kann, wenn die Barcode-Identifikation erfolgt.

Obwohl die Erfindung unter Bezugnahme auf bestimmte Ausführungsbeispiele beschrieben worden ist, ist es für einen Fachmann ersichtlich, dass verschiedene Änderungen ausgeführt werden können, ohne den Bereich der Erfindung zu verlassen. Folglich soll die Erfindung nicht auf die offenbarten Ausführungsbeispiele begrenzt sein, sondern soll alle Ausführungsbeispiele umfassen, die in den Bereich der beigefügten Patentansprüche fallen.

## Patentansprüche

1. Vorrichtung 100; 110; 120) zur Temperaturüberwachung einer kryokonservierten biologischen Probe, umfassend
a) einen Probenbehälter (1; 41) mit einem Aufnahmeraum (2) zur Aufnahme einer biologischen Probe (6), insbesondere ein Kryoröhrchen; und
b) mindestens eine Kammer 102; 112; 122), deren Innenraum mit dem Aufnahmeraum (2) nicht fluidisch verbunden ist und lediglich zum Teil mit einer Indikatorsubstanz (7) gefüllt ist, deren Schmelztemperatur in einem Bereich von -20 °C bis -140 °C liegt,
wobei die Kammer eine Barriere 54; 54b; 54c; 103; 113; 123) aufweist, die bewirkt, dass, wenn sich Indikatorsubstanz (7) in einem ersten Teilbereich (52a; 102a) der Kammer im flüssigen Aggregatszustand befindet, diese Indikatorsubstanz (7) verzögert in einen zweiten Teilbereich (52b; 102b) der Kammer gelangt; wobei die Vorrichtung so ausgestaltet ist, Var
iante i):
dass die Indikatorsubstanz (7) in einem Behältnis (105; 115) gelagert ist, das die Indikatorsubstanz (7) im flüssigen Aggregatszustand dicht umschließt; und die Vorrichtung ein in Bezug auf das Behältnis (105; 115) beweglich geführtes Aktivierungsteil (104; 114; 124) aufweist, das von einer Ausgangsstellung in eine Aktivierungsstellung bringbar ist, wobei die Bewegung in die Aktivierungsstellung bewirkt, dass das Aktivierungsteil (104; 114; 124) durch mechanischen Druck (105; 115) das Behältnis an mindestens einer Stelle zerstört, insbesondere derart, dass das Behältnis durchlässig für die Indikatorsubstanz im flüssigen Zustand wird;
oder Variante ii):
dass die Barriere ein in Bezug auf die Indikatorsubstanz (7) im flüssigen Zustand permeables Trennelement (54; 54b; 54c) ist, das zwischen dem ersten Teilbereich (52a) und zweiten Teilbereich (52b) angeordnet ist; wobei das Trennelement ausgelegt ist, bei Abkühlung der Vorrichtung auf eine Lagertemperatur, die unterhalb der Schmeltemperatur der Indikatorsubstanz liegt, aufgrund der thermischen Kontraktion an mindestens einer Stelle (55a; 56a; 57a) zu reißen, so dass die Indikatorsubstanz im flüssigen Aggregatszustand über die durch die mindestens eine Rissstelle gebildete Öffnung (55b; 56b; 57b) von dem ersten Teilbereich in den zweiten Teilbereich gelangen kann.

2. Vorrichtung nach Anspruch gemäß Variante i), **dadurch gekennzeichnet, dass** die Barriere 103; 113; 123) ein in dem zweiten Teilbereich angeordnetes und an den ersten Teilbereich angrenzendes Material mit flüssigkeitsaufnehmender Struktur ist.

3. Vorrichtung nach Anspruch 1 oder 2 gemäß Variante i), **dadurch gekennzeichnet, dass** das Behältnis als Plastikkissen (105) oder als Glaskugel (115) ausgeführt ist.

4. Vorrichtung nach einem der vorhergehenden Ansprüche, **dadurch gekennzeichnet, dass** der Probenbehälter einen Deckel (101; 111) zum Verschließen des Aufnahmeraums (2) aufweist, wobei die mindestens eine Kammer in den Deckel integriert ist.

5. Vorrichtung (100; 110) nach den Ansprüchen 2 und 4, **dadurch gekennzeichnet, dass** der Deckel (101) einen auf den Probenbehälter (1) aufsteckbaren und/oder aufschraubbaren Grundkörper (106), insbesondere mit H-förmigem Querschnitt, aufweist, wobei der Grundkörper (106) zur Ausbildung der mindestens einen Kammer eine Ausnehmung (102) aufweist, in der das Behältnis (105; 115) mit der Indikatorsubstanz (7) und das Material (103; 113) mit flüssigkeitsaufnehmender Struktur angeordnet sind, wobei das Aktivierungsteil (104; 114), insbesondere als ein Einschraubteil, am Grundkörper (103) beweglich in Richtung des Behältnisses (105; 115) geführt ist.

6. Vorrichtung nach Anspruch 4, **dadurch gekennzeichnet, dass** der Deckel einen mit einem oberen Endbereich des Aufnahmeraums in Eingriff stehenden Schaft aufweist und dass die mindestens eine Kammer in den Schaft integriert ist.

7. Vorrichtung (120) nach einem der vorhergehenden Ansprüche 1 bis 3, **dadurch gekennzeichnet, dass** die mindestens eine Kammer in einen Bodenbereich des Probenbehälters (1) integriert ist.

8. Vorrichtung (120) nach Anspruch 7 gemäß Variante i), **dadurch gekennzeichnet, dass** ein Bodenbereich des Probenbehälters (1) zur Ausbildung der mindestens einen Kammer eine Ausnehmung(122) aufweist, in der das Behältnis (105; 115) mit der Indikatorsubstanz (7) und das Material (103; 113) mit flüssigkeitsaufnehmender Struktur angeordnet sind, wobei die Ausnehmung durch ein Bodenteil (125), in dem das Aktivierungsteil beweglich geführt ist, verschlossen wird, wobei das Bodenteil vorzugsweise einen maschinenlesbaren und/oder optoelektronisch lesbaren Code, insbesondere einen Barcode oder 2D-Code, aufweist.

9. Vorrichtung (120) nach Anspruch 8, **dadurch gekennzeichnet,**
a) **dass** die Aktivierungsposition durch einen durch das Bodenteil gebildeten Anschlag (129) festgelegt ist, bis zu dem das Aktivierungsteil in das Bodenteil (129) hineingedrückt werden kann; und/oder
b) **dass** das Bodenteil (125) fest mit dem Bodenbereich des Probenbehälters verbunden ist, insbesondere verklebt, verschmolzen, verschweißt oder anderweitig fest mit dem Bodenbereich des Probenbehälters fixiert ist.

10. Vorrichtung nach einem der vorhergehenden Ansprüche, **dadurch gekennzeichnet, dass** eine Wandung des zweiten Teilbereichs der Kammer und/oder eine Wandung des ersten Teilbereichs der Kammer
a) eine Skala (24) aufweist, die einen Füllstand der Indikatorsubstanz (7) im jeweiligen Teilbereich oder eine Zeitdauer der Überschreitung der Schmelztemperatur anzeigt; und/oder
b) an mindestens einer Stelle transparent oder semi-transparent ist.

11. Vorrichtung nach einem der Ansprüche 2 bis 10, **dadurch gekennzeichnet, dass** eine Struktur und/oder eine Zusammensetzung des Materials mit flüssigkeitsaufnehmender Struktur so ausgebildet ist, dass eine Diffusionsgeschwindigkeit der Indikatorsubstanz in dem Material nichtlinear mit zunehmenden Abstand vom ersten Teilbereich abnimmt.

12. Vorrichtung nach einem der vorherigen Ansprüche gemäß Variante ii), **dadurch gekennzeichnet, dass** das in Bezug auf die Indikatorsubstanz (7) im flüssigen Zustand permeable Trennelement als poröse Trennwand, Membran, Folie, Haut oder Kapillarsystem ausgeführt ist.

13. Vorrichtung nach Anspruch 12, **dadurch gekennzeichnet, dass** das Trennelement (54; 54b; 54c) mindestens eine Sollbruchstelle (55a; 56a; 57a) aufweist, an der das Trennelement bei der Abkühlung der Vorrichtung auf die Lagertemperatur reißt.

14. Vorrichtung nach einem der vorhergehenden Ansprüche gemäß Variante ii), **dadurch gekennzeichnet, dass** in dem zweiten Teilbereich
a) ein Gas vorhanden ist, oder
b) eine Substanz, die einen niedrigeren Schmelzpunkt als die Indikatorsubstanz aufweist, vorhanden ist.

15. Vorrichtung nach einem der vorhergehenden Ansprüche, **dadurch gekennzeichnet, dass** eine Außenwand der Kammer eine verschließbare Öffnung (14) zum ersten Teilbereich (12a) aufweist, zum Befüllen des ersten Teilbereichs (12a) mit einer Indikatorsubstanz (7).

16. Vorrichtung nach einem der Ansprüche 1 bis 3 und 9 bis 15, **dadurch gekennzeichnet,**
a) **dass** die mindestens eine Kammer durch einen Behälter (31) gebildet ist; und
b) **dass** an einer Außenwand des Probenbehälters (1) eine Aufnahme (34), beispielsweise eine Hülse oder Einstecktasche, befestigt ist, in die der Behälter (31) zur Halterung am Probenbehälter (1) einsteckbar und/oder eingesteckt ist.

17. Vorrichtung nach einem der Ansprüche bis 3 und 9 bis 15, **dadurch gekennzeichnet,**
a) **dass** der Probenbehälter ein Kryoröhrchen (1) ist; und
b) **dass** die mindestens eine Kammer durch ein doppelwandiges Aufsteckteil (21) gebildet ist, das auf eine äußere Mantelfläche des Kryoröhrchens (1) aufsteckbar ist und dieses im aufgesteckten Zustand dabei zumindest teilweise umgreift.

18. Vorrichtung nach Anspruch 17, **dadurch gekennzeichnet, dass** der Probenbehälter mit dem Aufsteckteil verklebt, verschmolzen oder anderweitig fest fixiert ist.

19. Vorrichtung nach einem der Ansprüche bis 3 und 9 bis 15, **dadurch gekennzeichnet, dass** der Probenbehälter ein Beutel (41) zur Ablage von Blutproben oder Stammzellen ist, der in einer Kassette (42) gehaltert ist, wobei die mindestens eine Kammer durch einen Behälter (44; 45, 46) gebildet ist, der
a) an einer Außenseite des Beutels befestigt ist,
b) freischwimmend im Innern des Beutels vorhanden ist oder
c) an der Kassette befestigt ist.

20. Verfahren zur Temperaturüberwachung von kryokonservierten Proben, umfassend die Schritte:
a) Bereitstellen einer Vorrichtung zur Temperaturüberwachung nach einem der vorhergehenden Ansprüche, die mindestens eine Indikatorsubstanz im gefrorenen Zustand im ersten Teilbereich der Kammer enthält, wobei der Aufnahmeraum eine kroykonservierte Probe enthält (S1);
b) gekühltes Lagern der Vorrichtung zur Kryokonservierung (S2), wobei bei einer Vorrichtung gemäß Variante i) nach Erreichen der Lagertemperatur die Aktivierung der Vorrichtung erfolgt;
c) Prüfen, ob sich zu einem späteren Zeitpunkt im zweiten Teilbereich der Kammer Indikatorsubstanz befindet (S3).

21. Verfahren nach Anspruch 20, **dadurch gekennzeichnet, dass** eine Kenngröße bestimmt wird, die ein Maß der in den zweiten Teilbereich der Kammer übergegangenen Menge an Indikatorsubstanz und/oder ein Maß für die im ersten Teilbereich der Kammer befindlichen Menge an Indikatorsubstanz angibt.

22. Verfahren nach Anspruch 20 oder 21, **dadurch gekennzeichnet, dass** eine Substanz als Indikatorsubstanz (7) ausgewählt wird, deren Schmelztemperatur oder deren Schwellentemperatur, bei der die Viskosität der geschmolzenen Indikatorsubstanz einen bestimmten Zielwert unterschreitet, einer vorbestimmten Grenztemperatur, deren Überschreiten überwacht werden soll, entspricht.

23. Verfahren nach einem dervorhorgohondon Ansprüche 20 bis 22, **dadurch gekennzeichnet,**
a) **dass** die bereitgestellte Vorrichtung zur Temperaturüberwachung einen Deckel gemäß einem der Ansprüche bis 6, einen Bodenbereich gemäß einem der Ansprüche 7 bis 9 oder ein Trennelement gemäß der Variante ii) aufweist;
b) **dass** zur Überprüfung der Funktionstüchtigkeit der Vorrichtung zur Temperaturüberwachung zumindest einer der folgenden Prüfschritte durchgeführt wird:
b1) Prüfen, ob der zweite Teilbereich der mindestens einen Kammer nach dem Befüllen des ersten Teilbereichs mit Indikatorsubstanz und vor dem Einfrieren der Indikatorsubstanz frei von Indikatorsubstanz ist;
b2) Prüfen, ob der zweite Teilbereich der mindestens einen Kammer nach dem Gefrieren der im ersten Teilbereich der mindestens einen Kammer befindlichen Indikatorsubstanz und vor dem gekühlten Lagern der Vorrichtung zur Kryokonservierung frei von Indikatorsubstanz ist; oder
b3) falls eine kryokonservierte Probe zur Nutzung entnommen wird und falls sich im zweiten Teilbereich der Kammer dann keine Indikatorsubstanz befindet, Prüfen, ob die Abdeckung ordnungsgemäß in den permeablen zweiten Zustand übergegangen ist oder ob das Trennelement der mindestens einen Kammer ordnungsgemäß gerissen ist.

24. Vorrichtung oder Verfahren nach einem der vorhergehenden Ansprüche, **dadurch gekennzeichnet, dass** die Indikatorsubstanz mindestens einen Alkohol, welcher aus der Gruppe, die Octan-l-ol, Nonan-l-ol, Propan-1,2-diol, Propan-1,3-diol, Butan-1,2-diol, Butan-1,3-diol, Butan-2-ol, Pentan-1,5-diol, Pentan-l-ol, Cyclopentanol, Benzylalkohol umfasst, ausgewählt ist, sowie optional mindestens einen Farbstoff umfasst.

25. Vorrichtung oder Verfahren nach Anspruch 24, **dadurch gekennzeichnet, dass** der Farbstoff aus der Gruppe ausgewählt ist, welche Triphenylmethanfarbstoffe, Rhodaminfarbstoffe, insbesondere Xanthene, Azofarbstoffe sowie Phenazin- und Phenothiazinfarbstoffe umfasst.

26. Vorrichtung oder Verfahren nach Anspruch 24 oder 25, **dadurch gekennzeichnet, dass** die Indikatorsubstanz mindestens zwei Alkoholkomponenten, welche aus der Gruppe, die Octan-l-ol, Nonan-l-ol, Propan-1,2-diol, Propan-1,3-diol, Butan-1,2-diol, Butan-1,3-diol, Butan-2-ol, Pentan-1,5-diol, Pentan-l-ol, Cyclopentanol, Benzylalkohol umfasst, ausgewählt sind, umfasst und/oder die Indikatorsubstanz mindestens einen Farbstoff umfasst, der aus der Gruppe ausgewählt ist, welche Oil Red, Methylrot, Brillantgrün, Rhodamin B, Neutralrot, Methylenblau oder andere Farbstoffe, die zur Anfärbung von Zellen in der Zytologie verwendet werden, umfasst.

## Claims

1. Device (100; 110; 120) for temperature monitoring of a cryopreserved biological sample, comprising
a) a sample container (1; 41) with a receiving space (2) for receiving a biological sample (6), in particular a cryogenic tube; and
b) at least one chamber (102; 112; 122), the inner space of which is not fluidically connected to the receiving space (2) and is only partially filled with an indicator substance (7), the melting temperature of which lies in a range from -20°C to -140°C,
wherein the chamber has a barrier (54; 54b; 54c; 103; 113; 123) which brings about that, if indicator substance (7) is located in a first sub-region (52a; 102a) of the chamber in the liquid state, this indicator substance (7) passes into a second sub-region (52b; 102b) of the chamber in a delayed manner,
wherein the device is configured so
variant i):
that the indicator substance (7) is stored in a receptacle (105; 115) which tightly encloses the indicator substance (7) in the liquid state; and the device has an activation part (104; 114; 124) which is guided movably in relation to the receptacle (105; 115) and which can be moved from a starting position into an the activation position, wherein the movement into the activation position brings about that the activation part (104; 114; 124), as a result of mechanical pressure, destroys the receptacle (105; 115) at at least one point in particular in such a manner that the receptacle becomes permeable for the indicator substance in the liquid state;
or variant ii):
that the barrier is a separating element (54; 54b; 54c) which is permeable in relation to the indicator substance (7) in the liquid state and which is arranged between the first sub-region (52a) and second sub-region (52b); wherein the separating element is configured, in the case of cooling of the device to a storage temperature which lies below the melting temperature of the indicator substance, to tear at at least one point (55a; 56a; 57a) as a result of thermal contraction so that the indicator substance can pass in the liquid state via the opening (55b; 56b; 57b) formed by the at least one tear point from the first sub-region into the second sub-region.

2. Device according to Claim 1 in accordance to variant i), **characterised in that** the barrier (103; 113; 123) is a material, which is arranged in the second sub-region and adjoins the first sub-region, with a liquid-absorbing structure.

3. Device according to Claim 1 or 2 in accordance to variant i), **characterised in that** the receptacle is embodied as a plastic cushion (105) or as a glass ball (115).

4. Device according to any one of the preceding claims, **characterised in that** the sample container has a cover (101; 111) for closing off the receiving space (2), wherein the at least one chamber is integrated into the cover.

5. Device (100; 110) according to Claims 2 und 4, **characterised in that** the cover (101) has a base body (106), in particular with an H-shaped cross-section, which can be pushed and/or screwed onto the sample container (1), wherein the base body (106) has, for the formation of the at least one chamber, a recess (102) in which the receptacle (105; 115) with the indicator substance (7) and the material (103; 113) with a liquid-absorbing structure are arranged, wherein the activation part (104; 114), in particular as a screw-on part, on the base body (103) is guided movably in the direction of the receptacle (105; 115).

6. Device according to Claim 4, **characterised in that** the cover comprises a shaft which is in engagement with an upper end region of the receiving space and that the at least one chamber is integrated into the shaft.

7. Device (120) according to any one of the preceding Claims 1 to 3, **characterised in that** the at least one chamber of the device is integrated into a base region of the sample container (1).

8. Device (120) according to Claim 7 in accordance to variant i), **characterised in that** a base region of the sample container (1) has, for the formation of the at least one chamber, a recess (122) in which the receptacle (105; 115) with the indicator substance (7) and the material (103; 113) with a liquid-absorbing structure are arranged, wherein the recess is closed off by a base part (125) in which the activation part is guided movably, wherein the base part (125) preferably has a machine-readable and/or optoelectronically readable code, in particular a barcode or 2D code.

9. Device (120) according to Claim 8, **characterised in that**
a) the activation position is fixed by a stop (129) formed by the base part, up to which stop the activation part can be pushed into the base part (129); and/or
b) the base part (125) is connected fixedly to the base region of the sample container, in particular glued, melted, welded or otherwise fixed solidly to the base region of the sample container.

10. Device according to any one of the preceding claims, **characterised in that** a wall of the second sub-region of the chamber and/or a wall of the first sub-region of the chamber
a) comprises a scale (24) which displays a fill level of the indicator substance (7) in the respective sub-region or a duration of the exceeding of the melting temperature; and/or
b) is transparent or semi-transparent at at least one point.

11. Device according to any one of Claims 2 to 10, **characterised in that** a structure and/or a composition of the material with a liquid-absorbing structure is formed so that a diffusion speed of the indicator substance in the material reduces non-linearly with increasing distance from the first sub-region.

12. Device according to any of the preceding claims in accordance to variant ii), **characterised in that** the separating element which is permeable in relation to the indicator substance (7) in the liquid state is embodied as a porous separating wall, membrane, film, skin or capillary system.

13. Device according to Claim 12, **characterised in that** the separating element (54; 54b; 54c) has at least one predetermined breaking point (55a; 56a; 57a) at which the separating element tears during cooling of the device to the storage temperature.

14. Device according to any one of the preceding claims in accordance to variant ii), **characterised in that** in the second sub-region
a) a gas is present, or
b) a substance which has a lower melting point than the indicator substance is present.

15. Device according to any one of the preceding claims, **characterised in that** an outer wall of the chamber has a closable opening (14) to the first sub-region (12a) for filling the first sub-region (12a) with an indicator substance (7).

16. Device according to any one of Claims 1 to 3 and 9 to 15, **characterised in that**
a) the at least one chamber is formed by a container (31); and
b) there is fastened to an outer wall of the sample container (1) a receiver (34), for example, a sleeve or insertion pocket into which the container (31) can be inserted and/or is inserted for retention on the sample container (1).

17. Device according to any one of Claims 1 to 3 and 9 to 15, **characterised in that**
a) the sample container is a cryogenic tube (1); and
b) the at least one chamber is formed by a double-walled push-on part (21) which can be pushed onto an outer shell surface of the cryogenic tube (1) and at least partially engages around it in the pushed-on state.

18. Device according to Claim 17, **characterised in that** the sample container is glued, melted or fixed solidly in another manner to the push-on part.

19. Device according to any one of Claims 1 to 3 and 9 to 15, **characterised in that** the sample container is a bag (41) for the storage of blood samples or stem cells which is retained in a cassette (42), wherein the at least one chamber is formed by a container (44; 45, 46), which
a) is fastened to an outer side of the bag,
b) is present floating freely in the interior of the bag or
c) is fastened to the cassette.

20. Method for temperature monitoring of cryopreserved samples, comprising the steps:
a) providing a device for temperature monitoring according to any one of the preceding claims which contains at least one indicator substance in the frozen state in the first sub-region of the chamber, wherein the receiving space contains a cryopreserved sample (S1);
b) cooled storing of the device for cryopreservation (S2), wherein for a device in accordance to variant i) the activation of the device occurs after reaching the storage temperature;
c) checking whether indicator substance is located in the second sub-region of the chamber at a later point in time (S3).

21. Method according to Claim 20, **characterised in that** a parameter is determined which indicates a measure of the quantity of indicator substance which has moved into the second sub-region of the chamber and/or a measure of the quantity of indicator substance located in the first sub-region of the chamber.

22. Method according to Claim 20 or 21, **characterised in that** a substance, the melting temperature of which or the threshold temperature of which, at which the viscosity of the melted indicator substance undershoots a specific target value, corresponds to a predetermined threshold temperature, the exceeding of which should be monitored, is selected as the indicator substance (7).

23. Method according to any one of Claims 20 to 22, **characterised in that**
a) the provided device for temperature monitoring has a cover according to any one of Claims 4 to 6, a base region according to any one of Claims 7 to 9 or a separating element in accordance to variant ii);
b) at least one of the following test steps is carried out in order to check the functionality of the device for temperature monitoring:
b1) testing whether the second sub-region of the at least one chamber is free from indicator substance after filling of the first sub-region with indicator substance and prior to freezing of the indicator substance;
b2) testing whether the second sub-region of the at least one chamber is free from indicator substance after freezing of the indicator substance located in the first sub-region of the at least one chamber and prior to cooled storage of the device for cryopreservation; or
b3) if a cryopreserved sample is removed for use and if no indicator substance is then located in the second sub-region of the chamber, testing whether the covering has correctly transferred into the permeable second state or whether the separating element of the at least one chamber is correctly torn.

24. Device or method according to any one of the preceding claims, **characterised in that** the indicator substance comprises at least one alcohol which is selected from the group which comprises octan-1-ol, nonan-1-ol, propane-1,2-diol, propane-1,3-diol, butane-1,2-diol, butane-1,3-diol, butan-2-ol, pentane-1,5-diol, pentan-1-ol, cyclopentanol, benzyl alcohol as well as optionally at least one dye.

25. Device or method according to Claim 24, **characterised in that** the dye is selected from the group which comprises triphenylmethane dyes, rhodamine dyes, in particular xanthene, azo dyes as well as phenazine and phenothiazine dyes.

26. Device or method according to Claim 24 or 25, **characterised in that** the indicator substance comprises at least two alcohol components which are selected from the group which comprises octan-1-ol, nonan-1-ol, propane-1,2-diol, propane-1,3-diol, butane-1,2-diol, butane-1,3-diol, butan-2-ol, pentane-1,5-diol, pentan-1-ol, cyclopentanol, benzyl alcohol and/or the indicator substance comprises at least one dye which is selected from the group which comprises oil red, methyl red, brilliant green, rhodamine B, neutral red, methylene blue or other dyes which are used to colour cells in cytology.

## Revendications

1. Dispositif (100 ; 110 ; 120) pour la surveillance de la température d'un échantillon biologique cryoconservé, comprenant
a) un récipient d'échantillons (1 ; 41) avec un espace de réception (2) pour recevoir un échantillon (6) biologique, en particulier un cryotube ; et
b) au moins un compartiment (102 ; 112 ; 122), dont l'espace intérieur n'est pas relié de manière fluidique à l'espace de réception (2) et est rempli seulement en partie avec une substance indicatrice (7), dont la température d'ébullition se situe dans une plage de - 20 °C à -140 °C,
dans lequel le compartiment présente une barrière (54 ; 54b ; 54c ; 103 ; 113 ; 123) qui a pour effet que, quand la substance indicatrice (7) se trouve dans une première zone partielle (52a ; 102a) du compartiment dans l'état d'agrégat liquide, ladite substance indicatrice (7) parvient de manière retardée dans une deuxième zone partielle (52b ; 102b) du compartiment ;
dans lequel le dispositif est configuré de telle sorte
variante i) :
que la substance indicatrice (7) est stockée dans un contenant (105 ; 115), qui renferme de manière étanche la substance indicatrice (7) dans l'état d'agrégat liquide ; et le dispositif présente une partie d'activation (104 ; 114 ; 124) guidée de manière mobile par rapport au contenant (105 ; 115), qui peut être amenée depuis une position de départ dans une position d'activation, dans lequel le déplacement dans la position d'activation a pour effet que la partie d'activation (104 ; 114 ; 124) détruit par une pression mécanique (105 ; 115) le contenant en au moins un emplacement, en particulier de telle manière que le contenant devient perméable à la substance indicatrice dans l'état liquide ;
ou variante ii) :
que la barrière est un élément de séparation (54 ; 54b ; 54c) perméable par rapport à la substance indicatrice (7) dans l'état liquide, qui est disposé entre la première zone partielle (52a) et la deuxième zone partielle (52b) ; dans lequel
l'élément de séparation est conçu pour se déchirer en raison de la contraction thermique en au moins un emplacement (55a ; 56a ; 57a) lors du refroidissement du dispositif à une température de stockage, qui est inférieure à la température de fusion de la substance indicatrice de telle sorte que la substance indicatrice peut parvenir, dans l'état d'agrégat liquide, par l'intermédiaire de l'ouverture (55b ; 56b ; 57b) formée par l'au moins une fissuration, depuis la première zone partielle dans la deuxième zone partielle.

2. Dispositif selon la revendication 1 selon la variante i), **caractérisé en ce que** la barrière (103 ; 113 ; 123) est un matériau disposé dans la deuxième zone partielle et jouxtant la première zone partielle avec une structure d'absorption de liquide.

3. Dispositif selon la revendication 1 ou 2 selon la variante i), **caractérisé en ce que** le contenant est réalisé en tant que coussin plastique (105) ou en tant que bille de verre (115).

4. Dispositif selon l'une quelconque des revendications précédentes, **caractérisé en ce que** le récipient d'échantillons présente un couvercle (101 ; 111) pour fermer l'espace de réception (2), dans lequel l'au moins un compartiment est intégré dans le couvercle.

5. Dispositif (100 ; 110) selon les revendications 2 et 4, **caractérisé en ce que** le couvercle (101) présente un corps de base (106) pouvant être emboîté et/ou vissé sur le récipient d'échantillons (1), en particulier avec une section transversale en forme de H, dans lequel le corps de base (106) présente pour réaliser l'au moins un compartiment un évidement (102), dans lequel le contenant (105 ; 115) avec la substance indicatrice (7) et le matériau (103 ; 113) avec une structure d'absorption de liquide sont disposés, dans lequel la partie d'activation (104 ; 114), est guidée, en particulier en tant qu'une partie à visser, de manière mobile en direction du contenant (105 ; 115) sur le corps de base (103).

6. Dispositif selon la revendication 4, **caractérisé en ce que** le couvercle présente une tige se trouvant en prise avec une zone d'extrémité supérieure de l'espace de réception et que l'au moins un compartiment est intégré dans la tige.

7. Dispositif (120) selon l'une quelconque des revendications précédentes 1 à 3, **caractérisé en ce que** l'au moins un compartiment est intégré dans une zone de fond du récipient d'échantillons (1).

8. Dispositif (120) selon la revendication 7 selon la variante i), **caractérisé en ce**
**qu'**une zone de fond du récipient d'échantillons (1) présente pour réaliser l'au moins un compartiment un évidement (122), dans lequel le contenant (105 ; 115) avec la substance indicatrice (7) et le matériau (103 ; 113) avec une structure d'absorption de liquide sont disposés, dans lequel l'évidement est fermé par une partie de fond (125), dans laquelle la partie d'activation est guidée de manière mobile, dans lequel la partie de fond présente de préférence un code lisible par une machine et/ou lisible de manière optoélectronique, en particulier un code à barres ou un code 2D.

9. Dispositif (120) selon la revendication 8,
**caractérisé en ce**
a) **que** la position d'activation est fixée par une butée (129) formée par la partie de fond, jusqu'à laquelle la partie d'activation peut être enfoncée dans la partie de fond (129) ; et/ou
b) **que** la partie de fond (125) est reliée de manière solidaire à la partie de fond du récipient d'échantillons, en particulier est collée, fondue, soudée ou bloquée autrement de manière solidaire avec la zone de fond du récipient d'échantillons.

10. Dispositif selon l'une quelconque des revendications précédentes, **caractérisé en ce qu'**une paroi de la deuxième zone partielle du compartiment et/ou une paroi de la première zone partielle du compartiment a) présentent une graduation (24), qui indique un niveau de remplissage de la substance indicatrice (7) dans la zone partielle respective ou une durée du dépassement de la température de fusion ; et/ou b) sont transparentes ou semi-transparentes en au moins un emplacement.

11. Dispositif selon l'une quelconque des revendications 2 à 10,
**caractérisé en ce**
**qu'**une structure et/ou une composition du matériau sont réalisées avec une structure d'absorption de liquide de telle sorte qu'une vitesse de diffusion de la substance indicatrice dans le matériau diminue de manière non linéaire au fur et à mesure que la distance par rapport à la première zone partielle augmente.

12. Dispositif selon l'une quelconque des revendications précédentes selon la variante ii), **caractérisé en ce que** l'élément de séparation perméable dans l'état liquide par rapport à la substance indicatrice (7) est réalisé en tant que paroi de séparation poreuse, membrane, film, enveloppe ou système capillaire.

13. Dispositif selon la revendication 12,
**caractérisé en ce que** l'élément de séparation (54 ; 54b ; 54c) présente au moins un emplacement de rupture théorique (55a ; 56a ; 57a), sur lequel l'élément de séparation se déchire lors du refroidissement du dispositif à la température de stockage.

14. Dispositif selon l'une quelconque des revendications précédentes selon la variante ii), **caractérisé en ce que** dans la deuxième zone partielle
a) un gaz est présent, ou
b) une substance, qui présente un point d'ébullition plus bas que la substance indicatrice, est présente.

15. Dispositif selon l'une quelconque des revendications précédentes, **caractérisé en ce qu'**une paroi extérieure du compartiment présente une ouverture fermable (14) menant vers la première zone partielle (12a) pour remplir la première zone partielle (12a) d'une substance indicatrice (7).

16. Dispositif selon l'une quelconque des revendications 1 à 3 et 9 à 15, **caractérisé en ce**
a) **que** l'au moins un compartiment est formé par un récipient (31) ; et
b) **qu'**est fixé sur une paroi extérieure du récipient d'échantillons (1) un logement (34), par exemple une douille ou une poche enfichable, dans laquelle le récipient (31) peut être enfiché et/ou est enfiché pour être maintenu sur le récipient d'échantillons (1).

17. Dispositif selon l'une quelconque des revendications 1 à 3 et 9 à 15, **caractérisé en ce**
a) **que** le récipient d'échantillons est un cryotube (1) ; et
b) **que** l'au moins un compartiment est formé par une partie à emboîter (21) à double paroi, qui peut être emboîtée sur une surface enveloppante extérieure du cryotube (1) et qui entoure ce faisant au moins en partie ce dernier dans l'état emboîté.

18. Dispositif selon la revendication 17,
**caractérisé en ce que** le récipient d'échantillons est collé, soudé ou bloqué autrement de manière solidaire avec la partie à emboîter.

19. Dispositif selon l'une quelconque des revendications 1 à 3 et 9 à 15,
**caractérisé en ce**
**que** le récipient d'échantillons est un sachet (41) pour déposer des échantillons de sang ou des cellules souches, qui est maintenu dans une cassette (42), dans lequel l'au moins un compartiment est formé par un récipient (44 ; 45, 46), qui
a) est fixé sur un côté extérieur du sachet,
b) est présent de manière flottante librement dans l'intérieur du sachet ou
c) est fixé sur la cassette.

20. Procédé pour la surveillance de la température d'échantillons cryoconservés, comprenant les étapes :
a) de fourniture d'un dispositif pour la surveillance de la température selon l'une quelconque des revendications précédentes, qui contient au moins une substance indicatrice dans l'état gelé dans la première zone partielle du compartiment, dans lequel l'espace de réception contient un échantillon cryoconservé (S1) ;
b) d'entreposage réfrigéré du dispositif pour la cryoconservation (S2), dans lequel l'activation du dispositif est effectuée pour un dispositif selon la variante i) après l'atteinte de la température de stockage ;
c) de contrôle si de la substance indicatrice se trouve à un moment ultérieur dans la deuxième zone partielle du compartiment (S3).

21. Procédé selon la revendication 20,
**caractérisé en ce qu'**est définie une grandeur caractéristique, qui indique une mesure de la quantité en substance indicatrice passée dans la deuxième zone partielle du compartiment et/ou une mesure de la quantité en substance indicatrice se trouvant dans la première zone partielle du compartiment.

22. Procédé selon la revendication 20 ou 21,
**caractérisé en ce qu'**est choisie en tant que substance indicatrice (7) une substance, dont la température de fusion ou dont la température de seuil, à laquelle la viscosité de la substance indicatrice fondue est inférieure à une valeur cible définie, correspond à une température limite prédéfinie, dont le dépassement doit être surveillé.

23. Procédé selon l'une quelconque des revendications 20 à 22, **caractérisé en ce**
a) **que** le dispositif fourni pour la surveillance de la température présente un couvercle selon l'une quelconque des revendications 4 à 6, une zone de fond selon l'une quelconque des revendications 7 à 9 ou un élément de séparation selon la variante ii) ;
b) **que** pour vérifier le bon fonctionnement du dispositif pour la surveillance de la température, au moins une des étapes de contrôle suivantes est mise en œuvre :
b1) contrôler si la deuxième zone partielle de l'au moins un compartiment est dépourvue de substance indicatrice après le remplissage de la première zone partielle avec la substance indicatrice ou avant la surgélation de la substance indicatrice ;
b2) contrôler si la deuxième zone partielle de l'au moins un compartiment est dépourvue de substance indicatrice après la congélation de la substance indicatrice se trouvant dans la première zone partielle de l'au moins un compartiment et avant l'entreposage réfrigéré du dispositif pour la cryoconservation ; ou
b3) si un échantillon cryoconservé est retiré aux fins de l'utilisation et si alors aucune substance indicatrice ne se trouve dans la deuxième zone partielle du compartiment, contrôler si le recouvrement est passé en bonne et due forme dans le deuxième état perméable ou si l'élément de séparation de l'au moins un compartiment est déchiré en bonne et due forme.

24. Dispositif ou procédé selon l'une quelconque des revendications précédentes, **caractérisé en ce que** la substance indicatrice comprend au moins un alcool, lequel est choisi parmi le groupe qui comprend l'octane-1-ol, le nonane-1-ol, le propane-1,2-diol, le propane-1,3-diol, le butane-1,2-diol, le butane-1,3-diol, le butane-2-ol, le pentane-1,5-diol, le pentane-1-ol, le cyclopentanol, l'alcool benzylique, et comprend également en option au moins un colorant.

25. Dispositif ou procédé selon la revendication 24, **caractérisé en ce que**
le colorant est choisi parmi le groupe, lequel comprend des colorants de triphénylméthane, des colorants de rhodamine, en particulier des xanthènes, des colorants azoïques ainsi que des colorants phénazine et phénothiazine.

26. Dispositif ou procédé selon la revendication 24 ou 25, **caractérisé en ce**
**que** la substance indicatrice comprend au moins deux composants d'alcool, lesquels sont choisis parmi le groupe qui comprend l'octane-1-ol, le nonane-1-ol, le propane-1,2-diol, le propane-1,3-diol, le butane-1,2-diol, le butane-1,3-diol, le butane-2-ol, le pentane-1,5-diol, le pentane-1-ol, le cyclopentanol, l'alcool benzylique, et/ou la substance indicatrice comprend au moins un colorant qui est choisi parmi le groupe, lequel comprend du Oil Red 0, du rouge de méthyle, du vert brillant, de la rhodamine B, du rouge neutre, du bleu de méthyle ou d'autres colorants qui sont utilisés pour colorer des cellules en cytologie.
